# EUROPÄISCHE PATENTSCHRIFT

(11) **EP 3 260 192 B1**
(45) Veröffentlichungstag und Bekanntmachung des Hinweises auf die Patenterteilung: **12.12.2018**
(21) Anmeldenummer: 17174247.1
(22) Anmeldetag: 02.06.2017
(51) Int. Cl.: B01F 11/00, B01F 15/00, B01F 15/02, A61B 17/88

(54) **LAGER- UND MISCHVORRICHTUNG ZUR HERSTELLUNG EINES KNOCHENZEMENTS, UND VERFAHREN ZUR VERMISCHUNG EINES KNOCHENZEMENTS**
STORAGE AND MIXING DEVICE FOR PRODUCING A BONE CEMENT AND METHOD FOR MIXING BONE CEMENT
DISPOSITIF DE STOCKAGE ET DE MÉLANGE DESTINÉ À PRODUIRE UN CIMENT OSSEUX ET PROCÉDÉ POUR MÉLANGER CIMENT OSSEUX

(30) Priorität: 08.06.2016 DE 102016110561
(43) Veröffentlichungstag der Anmeldung: 27.12.2017
(73) Patentinhaber: Heraeus Medical GmbH, 61273 Wehrheim (DE)
(72) Erfinder: Vogt, Sebastian, 99092 Erfurt (DE); Kluge, Thomas, 56179 Vallendar (DE)
(74) Vertreter: Schultheiss & Sterzel Patentanwälte PartG mbB

(56) Entgegenhaltungen:
- WO-A1-2012/174670

## Beschreibung

Die Erfindung betrifft eine Lager- und Mischvorrichtung für Zweikomponenten-Polymethylmethacrylat-Knochenzemente, die Lager- und Mischvorrichtung aufweisend eine Kartusche mit einem zylindrischen Innenraum.

Die Erfindung betrifft auch ein Verfahren zur Vermischung von Ausgangskomponenten eines Knochenzements, insbesondere eines Zweikomponenten-Polymethylmethacrylat-Knochenzements, mit einer solchen Lager- und Mischvorrichtung.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30.). PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement, der üblicherweise als Knochenzementteig bezeichnet wird. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Knochenzementteigs, bis dieser erstarrt.

Das am häufigsten in Polymethylmethacrylat-Knochenzementen verwendete Monomer ist Methylmethacrylat. Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Bestandteile der Redoxinitiatorsysteme zusammenwirken. Deshalb werden die Bestandteile des Redoxinitiatorsystems in den separaten Ausgangskomponenten so angeordnet, dass diese keine radikalische Polymerisation auslösen können. Die Ausgangskomponenten sind bei geeigneter Zusammensetzung lagerstabil. Erst bei Vermischung der beiden Ausgangskomponenten zu einem Zementteig reagieren die zuvor getrennt in den beiden Pasten, Flüssigkeiten oder Pulvern gelagerten Bestandteile des Redoxinitiatorsystems, wobei Radikale gebildet werden, welche die radikalische Polymerisation des wenigstens einen Monomers auslösen. Die radikalische Polymerisation führt dann unter Verbrauch des Monomers zu Bildung von Polymeren, wobei der Zementteig aushärtet.

PMMA-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, US 5 344 232 A. Bei den dargelegten Vakuum-Zementiersystemen ist es erforderlich, zur Erzeugung des Unterdrucks eine externe Vakuumpumpe anzuschließen. Diese werden im Allgemeinen mit Druckluft unter Nutzung des Venturi-Prinzips betrieben. Die für den Betrieb der Vakuumpumpen notwendige Druckluft wird entweder aus stationären Druckluftanlagen oder auch aus elektrisch betriebenen Kompressoren bezogen. Daneben ist es auch möglich, für die Vakuumerzeugung elektrisch betriebene Vakuumpumpen zu verwenden.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche geschlossenen Full-Prepacked-Mischsysteme wurden mit der EP 0 692 229 A1, der DE 10 2009 031 178 B3, der US 5 997 544 A, der US 6 709 149 B1, der DE 698 12 726 T2 und der US 5 588 745 A vorgeschlagen. Auch bei diesen Mischsystemen ist eine externe Vakuumquelle notwendig. Aus der EP 0 796 653 A2 ist eine Vorrichtung zur Herstellung von Knochenzement aus einem Knochenzementpulver und einer Monomerflüssigkeit in einer Kartusche bekannt, bei dem ein Vakuum mit einem anschließbaren Vakuumkolben gezogen wird. Nachteilig ist bei diesem System, dass nach dem Öffnen beziehungsweise Anschließen eines Monomerflüssigkeitsbehälters bereits Monomerflüssigkeit in das Knochenzementpulver eindringen und reagieren kann, bevor mit dem Vakuumkolben die restliche Monomerflüssigkeit in die Kartusche gesaugt wird. Dadurch können ausgehärtete Stücke Knochenzemente in dem Zementteig entstehen, wodurch die Anwendbarkeit des dadurch inhomogenen Knochenzementteigs erschwert wird und die mechanischen Eigenschaften des ausgehärteten Knochenzements negativ beeinflusst werden. Zudem ist durch die vielen notwendigen Schritte beim Anschließen der Teile der Vorrichtung und beim Herstellen des Vakuums die Anwendung der Vorrichtung relativ aufwendig und dadurch fehleranfällig.

Das Patent DE 10 2009 031 178 B3 offenbart eine Lager- und Mischvorrichtung als Full-Prepacked-Mischsystem, in dem die zur Herstellung des Knochenzementteigs notwendigen Ausgangskomponenten bereits in der Lager- und Mischvorrichtung gelagert sind und in der Lager- und Mischvorrichtung zusammengeführt und gemischt werden können. Die Lager- und Mischvorrichtung weist einen zweiteiligen Austragskolben zum Verschluss einer Zementkartusche auf. Dabei wird eine Kombination aus einem gasdurchlässigen Sterilisationskolben und einem gasundurchlässigen Dichtungskolben verwendet. Dieses Prinzip eines geschlossenen Vakuummischsystems wird beim dem geschlossenen Zementiersystem PALACOS® PRO verwirklicht, das von der Firma Heraeus Medical GmbH hergestellt und vertrieben wird.

Die WO 2012/174670 A1 offenbart eine Vorrichtung, gemäß dem Oberbegriff des Anspruchs 1, zum blasenarmen Mischen und Austragen eines Produkts, bei der die Ausgangskomponenten in eine Kammer zum Vermischen eingefüllt werden. Mit einer Feder sollen Gase aus dem Gemisch hinausgepresst werden. Nachteilig ist, dass die Vorrichtung nicht zum Lagern der Ausgangskomponenten geeignet ist.

In der WO 00/35506 A1 wird eine Vorrichtung vorgeschlagen, bei der Polymethylmethacrylat-Knochenzementpulver in einer Kartusche gelagert wird, wobei das Zementpulver das gesamte Volumen der Kartusche ausfüllt und die Zwischenräume zwischen den Partikeln des Zementpulvers ein solches Volumen hat, das dem Volumen der Monomerflüssigkeit entspricht, das zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird, wobei hierzu ein Vakuum an einem Vakuumanschluss an der Unterseite der Kartusche angelegt wird. Dadurch wird die Monomerflüssigkeit durch das Zementpulver gezogen, wobei die in den Zwischenräumen der Zementpartikel befindliche Luft durch die Monomerflüssigkeit verdrängt wird. Auf eine mechanische Durchmischung des gebildeten Zementteigs mit einem Rührer wird dabei verzichtet.

Nachteilig an diesem System ist, dass Zementpulver, die schnell mit der Monomerflüssigkeit anquellen, mit dieser Vorrichtung nicht angemischt werden können, weil die schnell anquellenden Zementpulverpartikel nach einem Eindringen der Monomerflüssigkeit in das Zementpulver von ungefähr 1 bis 2 cm eine gelartige Barriere bilden und die Migration der Monomerflüssigkeit durch das gesamte Zementpulver behindern. Weiterhin kann bei Vakuumeinwirkung nicht ausgeschlossen werden, dass nach vollständiger Durchdringung des Zementpulvers durch die Monomerflüssigkeit die Monomerflüssigkeit über den Vakuumanschluss abgesaugt wird. Dann steht nicht genügend Monomerflüssigkeit für die Aushärtung durch radikalische Polymerisation zur Verfügung beziehungsweise das Mischungsverhältnis wird ungewollt verändert und damit auch die Konsistenz des Knochenzements. Weiterhin ist es problematisch, dass die zwischen den Zementpulverpartikeln eingeschlossen Luft durch die Monomerflüssigkeit von oben nach unten verdrängt werden soll, weil die gegenüber der Monomerflüssigkeit spezifisch leichtere Luft auf Grund der Schwerkraft das Bestreben hat, im Zementpulver nach oben zu wandern und nicht nach unten in Richtung Vakuumanschluss zu migrieren.

Bei der Verwendung von Vakuummischsystemen zur Zementierung müssen externe Vakuumpumpen beigestellt werden. Diese Vakuumpumpen sind kostenintensiv und müssen nach der Anwendung gereinigt werden. Weiterhin sind Vakuumschläuche zur Verbindung der Vakuumpumpen mit den Vakuummischsystemen notwendig. Diese Vakuumschläuche müssen den Vakuummischsystemen beigelegt sein. Vor dem Mischen mit einem Vakuummischsystem muss daher zuerst die Vakuumpumpe im Operations-Saal (OP-Saal) aufgebaut und an eine Energiequelle, wie Druckluft oder an elektrischen Strom angeschlossen werden. Danach wird die Vakuumpumpe mit einem Vakuumschlauch mit dem Vakuummischsystem verbunden. Diese Montageschritte kosten wertvolle OP-Zeit und sind potentiell fehlerbehaftet. Die Vakuumpumpe und die Verbindungsleitungen zum Vakuummischsystem und zu externen Energiequellen und Versorgungsleitungen benötigen Platz und stellen potentielle Stolperfallen und Hindernisse dar, die den gelegentlich hektischen Ablauf während einer Operation stören können.

Ein interessantes Konzept wird mit der EP 1 886 647 A1 vorgeschlagen. Das Zementpulver ist dabei in einer evakuierten Kartusche gelagert und die Monomerflüssigkeit befindet sich in einem separaten Behälter. Beim Öffnen der unter Unterdruck stehenden Kartusche wird die Monomerflüssigkeit in die Kartusche gesaugt ohne dass Luft einströmt. Es entsteht ein von Lufteinschlüssen freier Knochenzementteig. Dieses Konzept erfordert es, dass die Kartusche während der Lagerung vor ihrer Verwendung vakuumdicht verschlossen bleibt und keine unsterile Luft eindringen kann. Die Kartusche muss dazu stabil hermetisch abgedichtet sein. Nachteilig ist hieran also, dass der Aufbau aufwendig ist und dass der Inhalt der Kartusche nach dem Einsaugen der Monomerflüssigkeit nicht von einem extern zu bedienenden Mischsystem durchmischt werden kann, da eine Durchführung für eine Mischstange oder ein Mischrohr nicht ohne weiteres dauerhaft vakuumdicht ist.

Die Aufgabe der Erfindung besteht also darin, die Nachteile des Stands der Technik zu überwinden. Insbesondere sollen die Nachteile der bekannten Vakuummischsysteme mit externer Vakuumquelle überwunden werden. Die Erfindung hat dabei unter anderem die Aufgabe, eine einfache geschlossene Lager- und Mischvorrichtung zu entwickeln, bei der Polymethylmethacrylat-Knochenzementpulver (Zementpulver) und Monomerflüssigkeit in separaten Kompartimenten gelagert und anschließend vermischt werden können. Vom medizinischen Anwender soll das Polymethylmethacrylat-Knochenzementpulver mit der Monomerflüssigkeit innerhalb der Lager- und Mischvorrichtung zusammengeführt und vermischt werden können, ohne dass beide Ausgangskomponenten mit dem medizinischen Anwender in Berührung kommen. Ein Kontakt des medizinischen Anwenders mit dem Polymethylmethacrylat-Knochenzementpulver und mit der Monomerflüssigkeit soll möglichst ausgeschlossen sein. Bei der zu entwickelnden Vorrichtung handelt es sich bevorzugt um ein Full-Prepacked-Mischsystem. Die Lager- und Mischvorrichtung soll so beschaffen sein, dass die Monomerflüssigkeit in das Polymethylmethacrylat-Knochenzementpulver ohne die Verwendung von durch Druckluft oder Kompressoren oder elektrisch angetriebenen externen Vakuumpumpen, transferiert werden kann. Wichtig ist ferner, dass die Lager- und Mischvorrichtung ohne externe Energiequellen, wie Druckluft, Vakuum oder elektrischer Strom, auch unter einfachsten äußeren Bedingungen funktionsfähig und zuverlässig die Herstellung von Knochenzementteig gewährleistet. Die Lager- und Mischvorrichtung soll ohne zusätzliche technische Ausrüstung autonom verwendungsfähig sein. Dabei soll die Lager- und Mischvorrichtung möglichst einfach und kostengünstig aufgebaut sein.

Die Lager- und Mischvorrichtung soll dahingehend vereinfacht sein, dass mit nur einem manuellen Bedienelement zuerst ein Monomerflüssigkeitsbehälter beziehungsweise eine Glasampulle oder ein Folienbeutel als Monomerflüssigkeitsbehälter geöffnet werden kann und dass danach, ohne Nutzung von extern erzeugtem Vakuum, ein Transfer der Monomerflüssigkeit in die Kartusche mit darin enthaltenem Zementpulver manuell vorgenommen werden kann. Eine Fehlbedienung soll konstruktiv möglichst ausgeschlossen werden.

Es soll ferner ein Verfahren bereitgestellt werden, das einen Monomerflüssigkeitstransfer und ein Mischen in Full-Prepacked-Mischsystemen ermöglicht und bei dem nach dem Öffnen des Monomerflüssigkeitsbehälters nur ein einziges Bedienelement bedient werden muss, um die Monomerflüssigkeit zu transferieren, einen Unterdruck in dem Mischraum (der Kartusche) zu erzeugen und mit dem Knochenzementpulver zu dem gewünschten Knochenzementteig zu mischen. Dabei soll die zu entwickelnde Lager- und Mischvorrichtung, hauptsächlich aus kostengünstigem Kunststoff gefertigt werden können.

Des Weiteren soll eine kostengünstig zu fertigende und zuverlässig funktionierende Lager- und Mischvorrichtung zum Mischen eines medizinischen Knochenzements und zur Lagerung der Ausgangskomponenten des Knochenzements sowie ein Verfahren zum Mischen des Knochenzements gefunden werden, bei dem eine möglichst einfache manuelle Bedienung zum Mischen der Ausgangskomponenten angewendet werden kann, möglichst ohne dass eine externe oder zusätzliche Energiequelle verwendet werden muss und ohne dass Lufteinschlüsse in dem Mischgut entstehen.

Die erste Ausgangskomponente des Polymethylmethacrylat-Knochenzements als Mischgut soll ein Pulver beziehungsweise Knochenzementpulver sein und die zweite Ausgangskomponente soll in Form einer Flüssigkeit, der Monomerflüssigkeit, vorliegen. Die beiden Ausgangskomponenten des Knochenzements sollen bevorzugt in dem Full-Prepacked-Mischsystem getrennt gelagert werden können und durch Anwendung der Lager- und Mischvorrichtung sicher zusammengeführt werden können.

Die Aufgaben der Erfindung werden gelöst durch eine Lager- und Mischvorrichtung für Zweikomponenten-Polymethylmethacrylat-Knochenzemente, die Lager- und Mischvorrichtung aufweisend
eine Kartusche mit einem zylindrischen Innenraum, wobei der zylindrische Innenraum an einer Vorderseite durch einen Kartuschenkopf mit einer geschlossenen Austragsöffnung begrenzt ist,
einen Kolben, der axial beweglich im zylindrischen Innenraum der Kartusche angeordnet ist und der von dem Kartuschenkopf beabstandet ist, wobei der Kolben umlaufend an der Innenwand des Innenraums anliegt, so dass der Kolben den Innenraum der Kartusche gasdicht in zwei Bereiche trennt,
eine pulverförmige erste Ausgangskomponente des Knochenzements, die in dem Innenraum zwischen dem Kolben und dem Kartuschenkopf enthalten ist, eine Durchführung, die im Kartuschenkopf oder in dem Zylindermantel der Kartusche zwischen dem Kolben und dem Kartuschenkopf angeordnet ist, wobei die Durchführung mit einer Fluidleitung verbunden ist, in der eine Monomerflüssigkeit als zweite Ausgangskomponente des Knochenzements enthalten ist oder in die eine Monomerflüssigkeit einfüllbar oder einleitbar ist,
einen Filter, der im Kartuschenkopf, in der Durchführung und/oder in der Fluidleitung angeordnet ist, wobei der Filter für die Monomerflüssigkeit durchlässig und für die erste Ausgangskomponente undurchlässig ist, wobei
der Kolben von einer der Vorderseite gegenüberliegenden Rückseite des zylindrischen Innenraums derart weit beabstandet ist, so dass durch eine Bewegung des Kolbens in Richtung der Rückseite ein Unterdruck in dem Innenraum der Kartusche zwischen dem Kolben und dem Kartuschenkopf erzeugbar ist, wobei mit dem Unterdruck die Monomerflüssigkeit aus der Fluidleitung in den Innenraum der Kartusche zu saugen ist.

Bevorzugt kann vorgesehen sein, dass die erste Ausgangskomponente ein Zementpulver ist.

Der Innenraum der Kartusche hat eine zylindrische Geometrie. Die zylindrische Form ist die einfachste, mit der sich der Innenraum der Kartusche realisieren lässt. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Die begrenzende Innenwand des Innenraums kann also ein Zylinder mit beliebiger Grundfläche sein und ebenso kann gegebenenfalls der Mantel der Kartusche ein Zylinder mit beliebiger Grundfläche sein, das heißt auch mit nicht kreisförmiger oder nicht runder Grundfläche.

Erfindungsgemäß wird jedoch eine zylindrische Geometrie mit rotationssymmetrischer und insbesondere kreisrunder Grundfläche für den Innenraum der Kartusche bevorzugt, da diese am einfachsten zu fertigen sind und der Kolben dann weniger leicht im Innenraum verkannten kann, wenn er axial im Innenraum bewegt wird. Zudem werden mögliche Undichtigkeiten zwischen der Innenwand des Innenraums und dem Kolben während der Bewegung des Kolbens unwahrscheinlicher.

Dass der Kolben im zylindrischen Innenraum der Kartusche axial beweglich ist bedeutet, dass der Kolben entlang der Zylinderachse des zylindrischen Innenraums axial beweglich ist.

Bevorzugt ist zwischen dem Kolben und der pulverförmigen ersten Ausgangskomponenten kein weiteres Bauteil vorgesehen. Die Rückseite des Innenraums bildet auch die Rückseite der Kartusche.

Mit dem Filter muss konsequent das Vordringen von der pulverförmigen ersten Ausgangskomponente und auch von Stäuben der ersten Ausgangskomponenten verhindert werden, da ansonsten das Pulver die Fluidleitung in kurzer Zeit verschließt, wenn die Monomerflüssigkeit mit der ersten Ausgangskomponente in Kontakt kommt und damit die Lager- und Mischvorrichtung unbrauchbar macht, das heißt, keine Monomerflüssigkeit mehr in die Kartusche überführt werden kann.

Bei erfindungsgemäßen Lager- und Mischvorrichtungen kann vorgesehen sein, dass der Kolben mit einem Stab oder mit einem anderen Kraftübertragungsmittel in dem Innenraum der Kartusche manuell in Richtung der Rückseite des Innenraums zu drücken oder zu ziehen ist, so dass im Innenraum der Kartusche zwischen dem Kolben und dem Kartuschenkopf ein Unterdruck entsteht, wobei vorzugsweise an dem Stab oder an dem Kraftübertragungsmittel ein Betätigungsmittel oder ein Griff zum Bedienen des Stabs oder des Kraftübertragungsmittels befestigt ist.

Hiermit kann der Unterdruck leicht und unmittelbar manuell erzeugt werden, was einerseits die Gefahr möglicher Fehlfunktionen reduziert und andererseits komplexe und kostenintensive Bauteile wie Motoren, Energiespeicher oder Steuerungen vermieden werden. Zudem kann so eine einfache und sichere Bedienbarkeit der Lager- und Mischvorrichtung gewährleistet werden.

Ferner kann vorgesehen sein, dass der Filter ein Porenfilter ist. Vorzugsweise kann auch vorgesehen sein, dass der Filter für Zementpulver undurchlässig ist.

Durch den Filter und insbesondere den Porenfilter wird erfindungsgemäß verhindert, dass Zementpulver beziehungsweise die pulverförmige erste Ausgangskomponente in die Fluidleitung bis zur Monomerflüssigkeit vordringen kann, dort mit der Monomerflüssigkeit vorzeitig reagiert, fest beziehungsweise gel-artig wird und dadurch die Fluidleitung verschließt.

Hierzu ist der Filter vorzugsweise im Kartuschenkopf, in der Durchführung oder in der Fluidleitung im Bereich der Durchführung angeordnet.

Bevorzugt ist erfindungsgemäß vorgesehen, dass der Kolben derart (beziehungsweise derart weit) von der Rückseite des zylindrischen Innenraums beabstandet ist, dass der Hubraum des Kolbens bis zu der Rückseite zumindest genauso groß ist, wie das Volumen der einzusaugenden Monomerflüssigkeit, vorzugsweise zumindest genauso groß ist, wie das Volumen der Fluidleitung und eines Monomerflüssigkeitsbehälters, in dem die Monomerflüssigkeit enthalten ist.

Hiermit wird sichergestellt, dass der im Innenraum er Kartusche zu erzeugende Unterdruck ausreicht, um die gewünschte Menge der Monomerflüssigkeit in den Innenraum der Kartusche zu saugen.

Mit einer Weiterbildung der erfindungsgemäßen Lager- und Mischvorrichtung kann auch vorgesehen sein, dass die Lager- und Mischvorrichtung einen von der Kartusche separaten Behälter aufweist, in dem die Monomerflüssigkeit enthalten ist, wobei der Behälter mit der Fluidleitung verbunden oder verbindbar ist, wobei vorzugsweise in dem Behälter eine geschlossene Glasampulle enthaltend die Monomerflüssigkeit angeordnet oder anzuordnen ist, wobei die Glasampulle innerhalb des Behälters aufbrechbar ist.

Hierdurch wird ein sogenanntes Full-Prepacked-Mischsystem bereitgestellt, bei dem alle Ausgangskomponenten, das heißt die beiden Ausgangskomponenten (Zementpulver als erste Ausgangskomponente und Monomerflüssigkeit als zweite Ausgangskomponente) bereits in der Lager- und Mischvorrichtung enthalten sind, darin gelagert werden können und innerhalb der Lager- und Mischvorrichtung gemischt werden können.

Es kann erfindungsgemäß vorgesehen sein, dass die Lager- und Mischvorrichtung Öffnungen aufweist, durch die Luft von außen in die Fluidleitung nachströmt. Es kann dabei bei erfindungsgemäßen Lager- und Mischvorrichtungen mit Behälter vorgesehen sein, dass die Öffnungen in dem Behälter für die Monomerflüssigkeit angeordnet sind, besonders bevorzugt in einem Deckel an der Rückseite des Behälters angeordnet sind.

Hiermit wird erreicht, dass Luft von außen in die Fluidleitung nachströmen kann und so die Monomerflüssigkeit nicht gegen ein Vakuum oder einen Unterdruck in die Kartusche gesaugt werden muss. Damit wird der Transfer der Monomerflüssigkeit in die Kartusche erleichtert.

Dabei kann vorgesehen sein, dass die Kartusche, der separate Behälter und die Fluidleitung mit einem gemeinsamen Standfuß verbunden sind, wobei der Behälter und die Fluidleitung fest mit dem Standfuß verbunden sind und die Kartusche lösbar mit dem Standfuß verbunden ist, bevorzugt die Kartusche über ein Gewinde an den Standfuß geschraubt ist oder über eine Rastung mit dem Standfuß verbunden ist.

Hiermit wird die Anwendung der Lager- und Mischvorrichtung vereinfacht. In Operationssälen ist meist eine ebene Unterlage, wie beispielsweise ein Tisch, vorhanden, so dass die Lager- und Mischvorrichtung mit Hilfe des Standfußes dort aufgestellt und angewendet werden kann, ohne dass die Lager- und Mischvorrichtung in der Hand gehalten werden müsste. Hierdurch wird die Anwendung der Lager- und Mischvorrichtung weiter vereinfacht.

Des Weiteren kann vorgesehen sein, dass in dem separaten Behälter ein Monomerflüssigkeitsbehälter enthalten ist, insbesondere ein Folienbeutel oder eine Glasampulle enthalten ist, der innerhalb des separaten Behälters zu öffnen ist, so dass die Monomerflüssigkeit aus dem geöffneten Monomerflüssigkeitsbehälter in die Fluidleitung fließt, wobei vorzugsweise am Behälter eine von außen bedienbare Öffnungsvorrichtung zum Öffnen des Monomerflüssigkeitsbehälters angeordnet ist.

Hierdurch wird ein sogenanntes Full-Prepacked-Mischsystem bereitgestellt, bei dem alle Ausgangskomponenten, das heißt die beiden Ausgangskomponenten (Zementpulver als erste pulverförmige Ausgangskomponente und Monomerflüssigkeit als zweite Ausgangskomponente) bereits in der Lager- und Mischvorrichtung enthalten sind, darin gelagert werden können und innerhalb der Lager- und Mischvorrichtung gemischt werden können. Die Glasampulle oder der Folienbeutel, der vorzugsweise mit Aluminium beschichtet ist, sind zur langfristigen Lagerung der Monomerflüssigkeit als zweiter Ausgangskomponente zur Herstellung des PMMA-Knochenzementteigs besonders gut geeignet. Die Anwendung der Lager- und Mischvorrichtung wird so vereinfacht und erleichtert.

Damit die Monomerflüssigkeit ohne zusätzliche Krafteinwirkung fließen kann, muss die Lager- und Mischvorrichtung bestimmungsgemäß aufgestellt werden, damit die Gravitation die gewünschte Flussrichtung bewirkt. Demzufolge sind die im Rahmen der vorliegenden Erfindung verwendeten Begriffe oben und unten sowie oberhalb und unterhalb immer in Bezug auf die bestimmungsgemäße Aufstellung der Lager- und Mischvorrichtung bezogen.

Es kann auch ein Monomerflüssigkeitsbehälter mit mehreren Kammern zur Aufbewahrung der Monomerflüssigkeit verwendet werden. Unter einem Monomerflüssigkeitsbehälter wird im Rahmen der vorliegenden Erfindung also auch eine Vielzahl von separaten einzelnen Teil-Monomerflüssigkeitsbehältern verstanden, die in den separaten Behälter eingebracht werden können oder sind und die mit der Öffnungsvorrichtung geöffnet werden können.

Es kann vorgesehen sein, dass in oder an der Fluidleitung zur Kartusche ein Sieb oder ein Filter angeordnet ist, mit dem Bruchstücke oder Fetzen des geöffneten Monomerflüssigkeitsbehälters zurückgehalten werden können. Bevorzugt befindet sich das Sieb oder der Filter in dem Behälter direkt unterhalb des Monomerflüssigkeitsbehälters.

Mit einer Weiterbildung der Erfindung zur Ausnutzung der Gravitation als Antrieb für den Fluss der Monomerflüssigkeit in die Fluidleitung wird vorgeschlagen, dass der separate Behälter für den Monomerflüssigkeitsbehälter oberhalb der Fluidleitung zur Kartusche angeordnet ist. Dadurch kann die Monomerflüssigkeit aus dem Monomerflüssigkeitsbehälter nach dem Öffnen des Monomerflüssigkeitsbehälters aufgrund der Gravitation in die Fluidleitung fließen.

Gemäß einer besonders bevorzugt Weiterentwicklung der vorliegenden Erfindung kann vorgesehen sein, dass in dem Innenraum der Kartusche zwischen dem Kolben und dem Kartuschenkopf eine Mischeinrichtung angeordnet ist, mit der die erste Ausgangskomponente mit der Monomerflüssigkeit im Innenraum der Kartusche zu mischen ist.

Hiermit wird erreicht, dass die erste Ausgangskomponente mit der Monomerflüssigkeit im Innenraum der Kartusche gut durchmischt werden kann, um einen möglichst homogenen Knochenzementteig zu erzeugen. Bevorzugt kann die Mischeinrichtung manuell von außen bedient werden.

Bei Lager- und Mischvorrichtungen mit einer Mischeinrichtung kann vorgesehen sein, dass die Mischeinrichtung über einen Mischstab von außerhalb der Kartusche bedienbar ist, wobei der Mischstab gasdicht durch den Kartuschenkopf geführt ist sowie drehbar und in axialer Richtung beweglich ist, vorzugsweise der Mischstab durch die Austragsöffnung geführt ist.

Hiermit kann die Mischeinrichtung einfach und kraftvoll von außerhalb der Lager- und Mischvorrichtung manuell bedient werden. Zudem sind durch den Widerstand gegen die Bewegung der Mischeinrichtung die Konsistenz des Knochenzementteigs und damit die Einsatzfähigkeit des Knochenzementteigs durch den Anwender gut abzuschätzen.

Es kann erfindungsgemäß vorgesehen sein, dass im Bereich des Kartuschenkopfs außen an der Kartusche ein Befestigungsmittel, insbesondere ein Außengewinde, vorgesehen ist. Daran kann ein separates Austragsrohr befestigt werden. Bevorzugt wird jedoch ein hohler rohrförmiger Mischstab verwendet, mit dem die Mischeinrichtung bedienbar ist und mit dem der Kolben in dem Innenraum in Richtung der Rückseite des Innenraums gedrückt werden kann, um den Unterdruck im Innenraum zu erzeugen, wobei der rohrförmige Mischstab nach Entfernen eines Verschlusses aus dem rohrförmigen Mischstab als Austragsrohr verwendet werden kann. Dann ist kein Außengewinde mehr notwendig, wobei das Außengewinde auch zur Befestigung der Kartusche in einer Austragsvorrichtung beziehungsweise Auspressvorrichtung zum Vortreiben des Kolbens in Richtung Kartuschenkopf verwendet werden kann, um den fertig gemischten Knochenzementteig auszupressen und zu applizieren. Um die Austragsöffnung zu schließen ist dazu im Inneren des hohlen Mischstabs ein herausziehbarer Kern angeordnet, der den rohrförmigen Mischstab nach außen verschließt.

Ferner kann vorgesehen sein, dass der Kolben mit dem Mischstab in Richtung der Rückseite des Innenraums der Kartusche zu drücken ist.

Hiermit kann der Kolben mit der gleichen Vorrichtung, die auch zum Antreiben der Mischeinrichtung verwendet wird, nämlich mit dem Mischstab, angetrieben werden, um den Unterdruck im Innenraum der Kartusche zu erzeugen. Hiermit wird für beide Funktionen nur eine gemeinsame Vorrichtung benötigt und die Lager- und Mischvorrichtung benötigt nur eine abzudichtende Durchführung hierzu.

Mit einer Weiterbildung der vorliegenden Erfindung wird vorgeschlagen, dass der Mischstab ein Austragsrohr ist, in dem ein manuell entfernbarer Kern angeordnet ist, so dass der gemischte Knochenzementteig aus dem Innenraum der Kartusche durch das Austragsrohr ohne den Kern auszutreiben ist.

Hierzu wird das Austragsrohr, vorzugsweise zuvor, bis zu einem Anschlag aus dem Innenraum der Kartusche herausgezogen. Durch die Verwendung des Mischstabs als Austragsrohr wird kein zusätzliches oder separates Austragsrohr benötigt, das an der Lager- und Mischvorrichtung zu befestigen wäre. Der Mischstab ist zu diesem Zweck gegen den Kartuschenkopf abgedichtet und axial beweglich und drehbar in der Austragsöffnung beziehungsweise durch die Austragsöffnung geführt.

Alternativ kann vorgesehen sein, dass die Lager- und Mischvorrichtung ein separates Austragsrohr mit einem statischen Mischer aufweist, das an der Kartusche zu befestigen ist, vorzugsweise an dem Befestigungsmittel an der Kartusche zu befestigen ist, wobei besonders bevorzugt an dem Austragsrohr ein zum Außengewinde an der Kartusche passendes Innengewinde vorgesehen ist und/oder Elemente eines Bajonettverschlusses und/oder Rastelemente eines Rastverschlusses vorgesehen sind.

Es kann auch vorgesehen sein, dass im Innenraum der Kartusche an der Rückseite ein Anschlag vorgesehen ist, der die Bewegung des Kolbens in Richtung Rückseite begrenzt.

Hiermit wird verhindert, dass der Kolben über die Rückseite des Innenraums hinaus verschoben werden kann und ungewollt Knochenzementteig nach außen austritt.

Des Weiteren kann vorgesehen sein, dass der Kolben mindestens ein Rastelement besitzt, das mit einer Gegenrastung an der Innenseite der Kartusche im Bereich der Rückseite des Innenraums lösbar zu rasten ist.

Hierdurch wird der Kolben, wenn er bis zur Rückseite bewegt wurde, in Position gehalten. Dadurch werden ein ungewolltes Bewegen des Kolbens während der Mischung des Inhalts des Innenraums und eine Bewegung des Kolbens über die Rastposition hinaus vermieden. Um den fertig gemischten Knochenzementteig auszutragen, kann die Rastung einfach gelöst werden.

Bei erfindungsgemäßen Lager- und Mischvorrichtungen kann vorgesehen sein, dass die Kartusche, der Kartuschenkopf und der Kolben aus Kunststoff gefertigt sind, wobei als Kunststoffe Polyethylen-co-vinylalkohol (EVOH), Polybutylenterephthalat (PBT), Polyethylenterephthalat (PET) und Poly-methacrylsäuremethylester-co-acrylnitril bevorzugt werden.

Ferner kann erfindungsgemäß vorgesehen sein, dass im Kartuschenkopf eine gasdurchlässige Öffnung, insbesondere eine verschließbare gasdurchlässige Öffnung, angeordnet ist, wobei eine für Gase durchlässige und für Feststoffpartikel undurchlässige poröse Scheibe zwischen der ersten Ausgangskomponente und der Öffnung angeordnet ist, wobei bevorzugt die poröse Scheibe im Kartuschenkopf angeordnet ist.

Damit kann eine Sterilisierung der Lager- und Mischvorrichtung auch im Innenraum der Kartusche mit Hilfe eines sterilisierenden Gases, wie beispielsweise Ethylenoxid durchgeführt werden. Die Öffnung ist bevorzugt neben der Austragsöffnung angeordnet und kann mit einem Verschluss verschlossen sein oder geschlossen werden.

Es kann vorgesehen sein, dass im Kartuschenkopf eine gasdichte verschließbare Öffnung oberhalb der porösen Scheibe angeordnet ist, die eine Fläche von mindestens 20 mm² hat und die mit der umgebenden Atmosphäre verbunden ist.

Zur Vermeidung einer ungewollten Befüllung des Innenraums der Kartusche mit Monomerflüssigkeit kann vorgesehen sein, dass die Fluidleitung eine nach oben weisende Schlaufe aufweist, wobei der höchste Punkt der Schlaufe oberhalb der eines Reservoirs für die Monomerflüssigkeit beziehungsweise oberhalb der Verbindung zwischen dem separaten Behälter und einem verbreiterten Teil der Fluidleitung liegt.

Damit kann verhindert werden, dass die Monomerflüssigkeit beim Einfüllen in die Fluidleitung bereits in den Innenraum der Kartusche gelangt. Diese umgekehrt U-förmige Schlaufe der Fluidleitung erreicht, dass vor einer Bewegung des Kolbens in Richtung der Rückseite des Innenraums der Kartusche die Monomerflüssigkeit in der Fluidleitung bis zur Höhe des Scheitelpunkts in der Fluidleitung verbleibt, wodurch ein vorzeitiger Eintritt der Monomerflüssigkeit zum Zementpulver verhindert wird. Insbesondere bei hoch-viskosen Zementen kann ein vorzeitiger Kontakt von schon geringen Mengen der Monomerflüssigkeit mit dem Zementpulver zum Verkleben der Fluidleitung führen. Die Fluidleitung kann transparent oder transluzent sein, damit der Anwender den Monomerflüssigkeitstransfer visuell kontrollieren kann. Hierzu kann insbesondere ein Sichtfenster in der Lager- und Mischvorrichtung vorgesehen sein, durch das die Schlaufe mit dem höchsten Scheitelpunkt zu erkennen ist.

Es kann erfindungsgemäß vorgesehen sein, dass der Kolben ein Austragskolben ist, mit dem der gemischte Knochenzementteig durch Vortreiben des Austragskolbens in Richtung Kartuschenkopf aus der Kartusche durch die Austragsöffnung oder durch das Austragsrohr oder den hohlen Mischstab auszupressen ist.

Bevorzugt ist der Kartuschenkopf oben angeordnet und die Rückseite der Kartusche unten, wenn die Lager- und Mischvorrichtung bestimmungsgemäß aufgestellt ist.

Die der vorliegenden Erfindung zugrundeliegenden Aufgaben werden auch gelöst durch ein Verfahren zur Vermischung der Ausgangskomponenten eines Knochenzements, insbesondere eines Mehrkomponenten-Polymethylmethacrylat-Knochenzements, mit einer erfindungsgemäßen Lager- und Mischvorrichtung, gekennzeichnet durch die folgenden Schritte:
a) mit dem Filter wird ein Vordringen der ersten Ausgangskomponente in die Fluidleitung verhindert,
b) der Kolben wird in Richtung der Rückseite des zylindrischen Innenraums der Kartusche bewegt, wobei durch die Bewegung des Kolbens im Innenraum der Kartusche zwischen dem Kolben und dem Kartuschenkopf ein Unterdruck entsteht,
c) die Monomerflüssigkeit wird mit dem Unterdruck im Innenraum aus der Fluidleitung und durch den Filter in den Innenraum der Kartusche gesaugt,
d) die Monomerflüssigkeit und die erste Ausgangskomponente werden im Innenraum der Kartusche zu dem Knochenzementteig gemischt, und
e) der Knochenzementteig wird durch Vortreiben des Kolbens in Richtung des Kartuschenkopfs aus dem Innenraum der Kartusche durch die geöffnete Austragsöffnung ausgetrieben.

Die Schritte a), b) und c) können zumindest zeitweise gleichzeitig ablaufen, während die Schritte d) und e) chronologisch nacheinander und auch chronologisch nach den Schritten b) und c) erfolgen. Schritt a) bezieht sich auf eine Wirkung des Filters, die genaugenommen während des gesamten Verfahrens erhalten bleibt, aber zumindest zu Beginn des Verfahrens vorhanden sein muss. Da sich schon während der Bewegung ein Unterdruck im Innenraum der Kartusche aufbaut, kann dieser Unterdruck bereits ein Einsaugen der Monomerflüssigkeit in den Innenraum der Kartusche bewirken.

Es kann vorgesehen sein, dass (zeitlich gesehen) vor Schritt b) die Monomerflüssigkeit in die Fluidleitung eingeleitet wird, insbesondere nach Öffnen eines Monomerflüssigkeitsbehälters in der Lager- und Mischvorrichtung.

Hiermit wird das Verfahren für Full-Prepacked-Systeme anwendbar, da die Monomerflüssigkeit in einem dafür vorgesehenen Monomerflüssigkeitsbehälter, wie beispielsweise einer Glasampulle oder einem metallbeschichteten Folienbeutel auch längerfristig in der Lager- und Mischvorrichtung gelagert werden kann.

Bei erfindungsgemäßen Verfahren kann auch vorgesehen sein, dass in Schritt b) der Kolben mit einem Mischstab in Richtung der Rückseite der Kartusche gedrückt wird, wobei der Mischstab beweglich in einer gasdichten Durchführung im Kartuschenkopf gelagert ist, und in Schritt d) die Mischung der Monomerflüssigkeit mit der ersten Ausgangskomponente durch Bewegen einer mit dem Mischstab verbundenen Mischeinrichtung im Innenraum der Kartusche zwischen dem Kolben und dem Kartuschenkopf erfolgt, indem der Mischstab und damit die Mischeinrichtung bewegt wird. Die gasdichte Durchführung ist bevorzugt durch die Austragsöffnung realisiert.

Hierdurch wird der Mischstab, der zum Bewegen der Mischeinrichtung in dem Innenraum der Kartusche vorhanden ist, gleichzeitig zum Bewegen des Kolbens und damit zum Erzeugen des Unterdrucks im Innenraum der Kartusche verwendet. Besonders bevorzugt kann dieser Mischstab als Rohr ausgeführt sein und so zusätzlich auch noch als Austragsrohr zum Applizieren des gemischten Knochenzementteigs verwendet werden.

Dabei kann wiederum vorgesehen sein, dass zwischen Schritt d) und e) die Mischeinrichtung mit dem Mischstab zum Kartuschenkopf gezogen wird und ein Kern aus dem Mischstab entfernt wird, so dass der Mischstab ohne den Kern ein Austragsrohr bildet, durch das der gemischte Knochenzementteig in Schritt e) aus dem Innenraum der Kartusche ausgepresst wird.

Hiermit wird der Mischstab zusätzlich auch noch als Austragsrohr nutzbar und die Austragsöffnung kann zur Durchführung des Mischstabs genutzt werden.

Es wird ferner im Rahmen der vorliegenden Erfindung vorgeschlagen, dass vor Schritt e) die Kartusche von der Lager- und Mischvorrichtung getrennt wird und in eine Auspressvorrichtung eingesetzt wird, mit der in Schritt e) der Kolben mittels eines Stößels oder einer vortreibbaren Stange in Richtung des Kartuschenkopfs gedrückt wird, um den Knochenzementteig aus dem Innenraum der Kartusche auszutreiben.

Hiermit kann die Kartusche getrennt von der Lager- und Mischvorrichtung zum Applizieren des bereits fertig gemischten Knochenzementteigs verwendet werden. Dadurch wird die Handhabung während der Applikation vereinfacht.

Es kann des Weiteren vorgesehen sein, dass vor Schritt b) der Kartuschenkopf gasdicht verschlossen wird.

Hiermit wird sichergestellt, dass in dem Innenraum der Kartusche ein Unterdruck erzeugt werden kann, der zum Einsaugen der Monomerflüssigkeit verwendet wird. Gleichzeitig kann aber zuvor durch den geöffneten Kartuschenkopf das Innere der Kartusche mit Hilfe eines sterilisierenden Gases, wie beispielsweise Ethylenoxid, sterilisiert werden.

Es kann auch vorgesehen sein, dass in Schritt c) Luft von außerhalb der Lager- und Mischvorrichtung durch Öffnungen in einem Behälter für die Monomerflüssigkeit nachströmt.

Damit wird verhindert, dass das Einsaugen der Monomerflüssigkeit in die Kartusche durch einen entstehenden Unterdruck in dem Behälter für die Monomerflüssigkeit verhindert oder erschwert wird.

Erfindungsgemäße Lager- und Mischvorrichtungen und erfindungsgemäße Verfahren sind theoretisch auch mit einer Mehrzahl von Kartuschen umsetzbar, in denen eine jeweils ein beweglicher Kolben zum Erzeugen eines Unterdrucks angeordnet ist. Derartige Lösungen werden als im Rahmen der vorliegenden Erfindung liegend verstanden, da dort ja auch immer jeweils eine Kartusche erfindungsgemäß betrieben wird, beziehungsweise betrieben werden kann.

Der Erfindung liegt die überraschende Erkenntnis zugrunde, dass der Kolben, der auch zum Austragen des gemischten Knochenzementteigs aus der Kartusche verwendet wird, zuvor zum Erzeugen eines Unterdrucks und zum Einsaugen der Monomerflüssigkeit verwendet werden kann. Wenn der Mischstab zudem noch zur Bedienung beziehungsweise zur Bewegung des Kolbens zum Erzeugen des Unterdrucks verwendet wird, können hierbei zusätzliche Bauteile vermieden werden. Ferner kann der Mischstab auch als Austragsrohr verwendet werden, um die Anzahl der notwendigen Bauteile zu minimieren. Die Idee besteht dabei darin, dass der Kolben nicht bereits an der Rückseite des Innenraums der Kartusche anliegt, sondern von diesem beabstandet angeordnet ist. Der Hub des Kolbens bis zur Rückseite des Innenraums der Kartusche kann dann genutzt werden, um im Innenraum der Kartusche einen Unterdruck zu erzeugen, mit dem die Monomerflüssigkeit in den Innenraum der Kartusche gesaugt werden kann. Der gegebenenfalls verbleibende Unterdruck kann dann dazu genutzt werden, die Ausgangskomponenten unter Unterdruck miteinander zu mischen, um im entstehenden Knochenzementteig möglichst wenige Gas- beziehungsweise Lufteinschlüsse entstehen zu lassen.

Eine erfindungsgemäße Lager- und Mischvorrichtung für Polymethylmethacrylat-Knochenzement ist beispielsweise zusammengesetzt aus
a) einer zylinderförmigen Kartusche,
b) einem in der zylinderförmigen Kartusche axial beweglichen Kolben,
c) einem Kartuschenkopf mit einer Durchführung für einen Mischstab,
d) einem Mischstab, der durch die Durchführung des Kartuschenkopfs axial verschiebbar ist,
e) einer Mischeinrichtung, die mit dem im Innenraum der Kartusche angeordneten Ende des Mischstabs verbunden ist,
f) einem Betätigungsmittel, das am äußeren Ende des Mischstabs angeordnet ist,
g) mindestens einem außerhalb der Kartusche angeordneten Monomerflüssigkeitsbehälter,
h) einer Öffnungsvorrichtung für den Monomerflüssigkeitsbehälter,
i) einer Fluidleitung, die den mindestens einen Monomerflüssigkeitsbehälter mit dem von der Kartusche, dem Kartuschenkopf und dem Kolben gebildeten Hohlraum flüssigkeitsdurchlässig verbindet,
j) einem Zementpulver, das in dem von der Kartusche, dem Kartuschenkopf und dem Kolben gebildeten Hohlraum angeordnet ist,
k) wobei der Kolben derart axial in der Kartusche angeordnet ist, dass der von der Kartusche, dem Kartuschenboden und dem Kolben gebildete Hubraum ein Volumen gleich oder größer dem gesamten Volumen der Fluidleitung und des mindestens einen Monomerflüssigkeitsbehälters hat.

Die Durchführung für den Mischstab ist durch die Austragsöffnung realisiert, beziehungsweise wird vorliegend als solche bezeichnet, wenn der Mischstab als Austragsrohr verwendet wird. Das Betätigungsmittel kann beispielsweise ein Griff sein, mit dem der Mischstab manuell bedienbar ist.

Erfindungsgemäß kann dabei ein Fußteil vorgesehen sein, mit dem die Kartusche lösbar verbunden ist und das mit dem mindestens einem Monomerflüssigkeitsbehälter unlösbar verbunden ist.

Es kann ferner vorgesehen sein, dass im Kartuschenkopf eine für Gase und Flüssigkeiten durchlässige poröse Scheibe angeordnet ist, die mit einer gasdurchlässigen Durchführung verbunden ist und die den Hohlraum durchlässig für Gase und Flüssigkeiten und undurchlässig für Feststoffpartikel abschließt.

Im Kartuschenkopf kann eine gasdicht verschließbare Öffnung oberhalb der porösen Scheibe angeordnet sein, die mindestens eine Fläche von mindestens 20 mm² hat und die mit der umgebenden Atmosphäre verbunden ist.

Erfindungsgemäß kann des Weiteren vorgesehen sein, dass der Kolben mindestens ein Rastelement besitzt, das mit einem Gegenrastelement an der Innenseite der Kartusche oberhalb des Kartuschenbodens lösbar gerastet werden kann.

Erfindungsgemäß ist beispielsweise ein Verfahren zum Mischen und Austragen von Polymethylmethacrylat-Knochenzement. Das Verfahren ist durch folgende nacheinander ablaufende Schritte gekennzeichnet,
a) gasdichtes Verschließen des Kartuschenkopfs,
b) Öffnen des mindestens einen Monomerflüssigkeitsbehälters,
c) Drücken des Betätigungsmittels des Mischstabs in Richtung Kartuschenboden (Rückseite des Innenraums der Kartusche),
d) Verschieben des Kolbens durch den Mischstab in Richtung des Kartuschenbodens,
e) Erzeugen eines Unterdrucks in dem von der Kartusche, dem Kartuschenkopf und dem Kolben gebildeten Hohlraum (im Innenraum der Kartusche),
f) Transfer der Monomerflüssigkeit vom geöffneten Monomerflüssigkeitsbehälter durch die Fluidleitung in den Hohlraum durch den gebildeten Unterdruck,
g) Mischen des Zementpulvers mit der Monomerflüssigkeit durch manuelle Betätigung des Betätigungselements des Mischstabs durch axiale und drehende Bewegung der Mischeinrichtung,
h) Ziehen des Mischstabs zum Kartuschenkopf,
i) Entfernen eines Kerns aus dem hohlen Mischstab,
j) Einsetzen der Kartusche in eine Auspressvorrichtung,
k) Betätigung der Auspressvorrichtung, wobei der Kolben in Richtung des Kartuschenkopfs bewegt wird, und
l) Auspressen des gemischten Knochenzementteigs aus der Kartusche durch den hohlen Mischstab.

Im Folgenden wird ein weiteres Ausführungsbeispiel der Erfindung anhand von acht schematisch dargestellten Figuren erläutert, ohne jedoch dabei die Erfindung zu beschränken. Dabei zeigt:
Figur 1: eine schematische Querschnittansicht einer beispielhaften erfindungsgemäßen Lager- und Mischvorrichtung im Ausgangszustand;
Figur 2: eine schematische perspektivische Ansicht der Lager- und Mischvorrichtung nach Figur 1;
Figur 3: eine schematische Querschnittansicht der Lager- und Mischvorrichtung mit geöffnetem Monomerflüssigkeitsbehälter;
Figur 4: eine schematische Querschnittansicht der Lager- und Mischvorrichtung während der Bewegung des Kolbens zum Einsaugen der Monomerflüssigkeit;
Figur 5: eine schematische Querschnittansicht der Lager- und Mischvorrichtung mit eingesaugter Monomerflüssigkeit;
Figur 6: eine schematische Querschnittansicht der Lager- und Mischvorrichtung mit gemischtem Knochenzementteig;
Figur 7: eine schematische perspektivische Querschnittansicht der Lager- und Mischvorrichtung; und

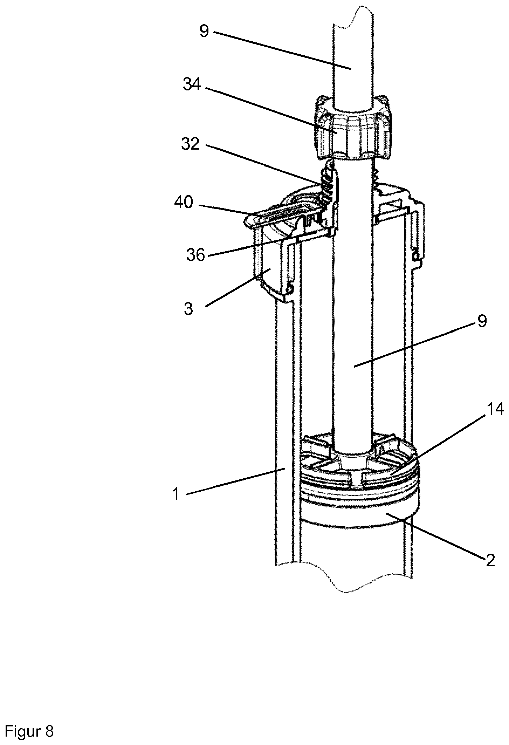

Figur 8: ein Ausschnitt einer schematischen perspektivischen Teilquerschnittansicht der Lager- und Mischvorrichtung.

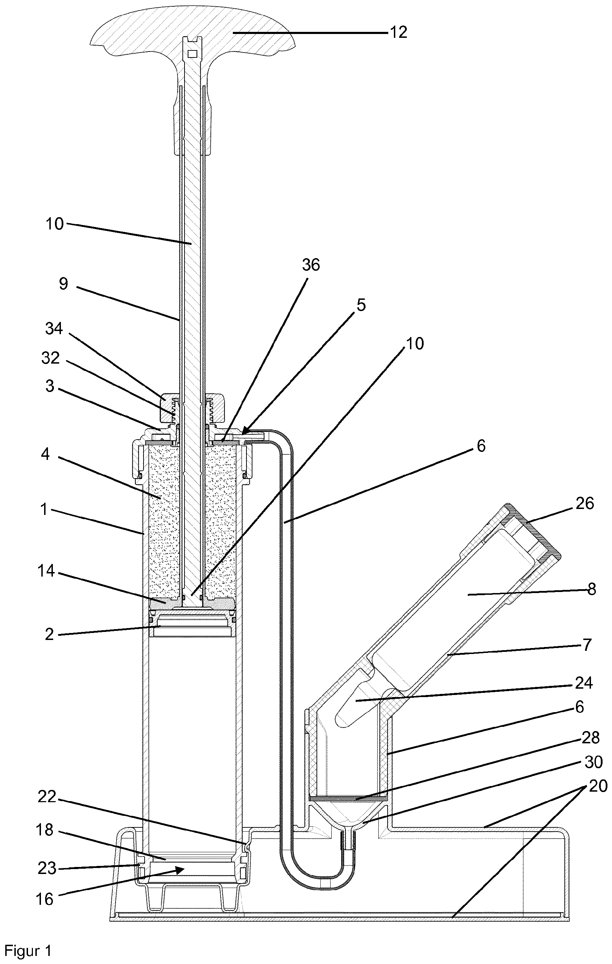

Die Figuren zeigen eine beispielhafte Ausführung einer erfindungsgemäßen Lager- und Mischvorrichtung. Figur 1 zeigt dabei eine schematische Querschnittansicht der beispielhaften erfindungsgemäßen Lager- und Mischvorrichtung im Ausgangszustand. Die Lager- und Mischvorrichtung umfasst eine Kartusche 1 mit einem zylindrischen Innenraum, in dem ein Kolben 2 linear axial beweglich angeordnet ist. An der Vorderseite ist der Innenraum beziehungsweise die Kartusche 1 durch einen Kartuschenkopf 3 nach Art eines Deckels oder einer Kappe verschlossen. Der Kartuschenkopf 3 ist gegen die Kartusche 1 mit einer umlaufenden Dichtung abgedichtet. In dem Innenraum der Kartusche 1 befindet sich zwischen dem Kolben 2 und dem Kartuschenkopf 3 ein Knochenzementpulver 4 als erste Ausgangskomponente 4 eines PMMA-Knochenzements.

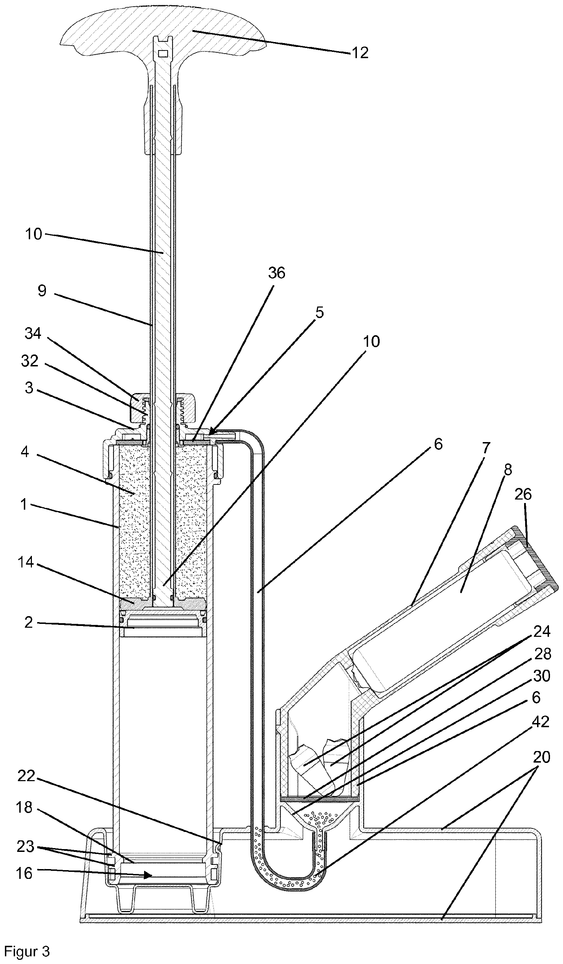

Der Kolben 2 schließt mit einer umlaufenden Gummidichtung gasdicht mit der Innenwand des Innenraums der Kartusche 1 ab. Dadurch kann durch eine Bewegung des Kolbens 2 vom Kartuschenkopf 3 weg im Innenraum der Kartusche 1 ein Unterdruck entstehen, der durch eine Durchführung 5 im Kartuschenkopf 3 zu einer Fluidleitung 6 geleitet wird, beziehungsweise auf die Fluidleitung 6 einwirkt. Die Fluidleitung 6 erstreckt sich zunächst parallel zur Achse der zylindrischen Kartusche 1 nach unten vom Kartuschenkopf 3 weg, vollführt dann eine Schleife und erstreckt sich dann wieder in die entgegengesetzte Richtung nach oben, wo die Fluidleitung 6 sich verbreitert. Der verbreiterte Teil der Fluidleitung 6 ist unterhalb eines zur Kartusche 1 separaten Behälters 7 angeordnet, in den eine Glasampulle 8 kopfüber eingesteckt ist. Die Glasampulle 8 ist in den geschnittenen Ansichten der Figuren 1 und 3 bis 6 nicht geschnitten dargestellt und ist in Figur 1 mit einer Monomerflüssigkeit 42 (siehe Figuren 3 bis 6) gefüllt. Die verbreiterte Fluidleitung 6 bildet eine Aufnahme für die Monomerflüssigkeit 42 aus der Glasampulle 8.

In dem Kartuschenkopf 3 befindet sich eine zentrale Austragsöffnung, durch die ein hohler beziehungsweise rohrförmiger Mischstab 9 hindurchgeführt ist. Der hohle Mischstab 9 ist in seinem Inneren mit einem Kern 10 verschlossen, der sich aus dem Mischstab 9 herausziehen lässt. Der Mischstab 9 ist mit einer umlaufenden Dichtung im Bereich der Austragsöffnung gegen den Kartuschenkopf 3 abgedichtet, so dass der Unterdruck im Innenraum der Kartusche 1 nicht (oder zumindest nicht schnell) Luft zwischen dem Kartuschenkopf 3 und dem Mischstab 9 in den Innenraum der Kartusche 1 nachziehen kann, auch wenn der Mischstab 9 in der Austragsöffnung gedreht und in Längsrichtung bewegt wird.

An dem vorderen Ende des Mischstabs 9 ist ein Griff 12 angeordnet, mit dem der Mischstab 9 manuell gegen die Kartusche 1 in Längsrichtung bewegt und axial gedreht werden kann. Zudem kann mit dem Griff 12 nach Lösen einer Rastung (nicht gezeigt) oder nach Überwinden eines Widerstands der Kern 10 aus dem Mischstab 9 herausgezogen werden.

Am Mischstab 9 ist im Innenraum der Kartusche 1 zwischen dem Kolben 2 und dem Kartuschenkopf 3 eine Mischeinrichtung 14 mit mehreren Mischflügeln 14 befestigt, so dass sich beim Drehen und Bewegen des Mischstabs 9 gegen die Kartusche 1 auch die Mischeinrichtung 14 im Innenraum der Kartusche 1 dreht und axial bewegt. Damit kann über den Mischstab 9 der Inhalt des Innenraums der Kartusche 1 mit Hilfe der Mischeinrichtung 14 mechanisch durchmischt werden.

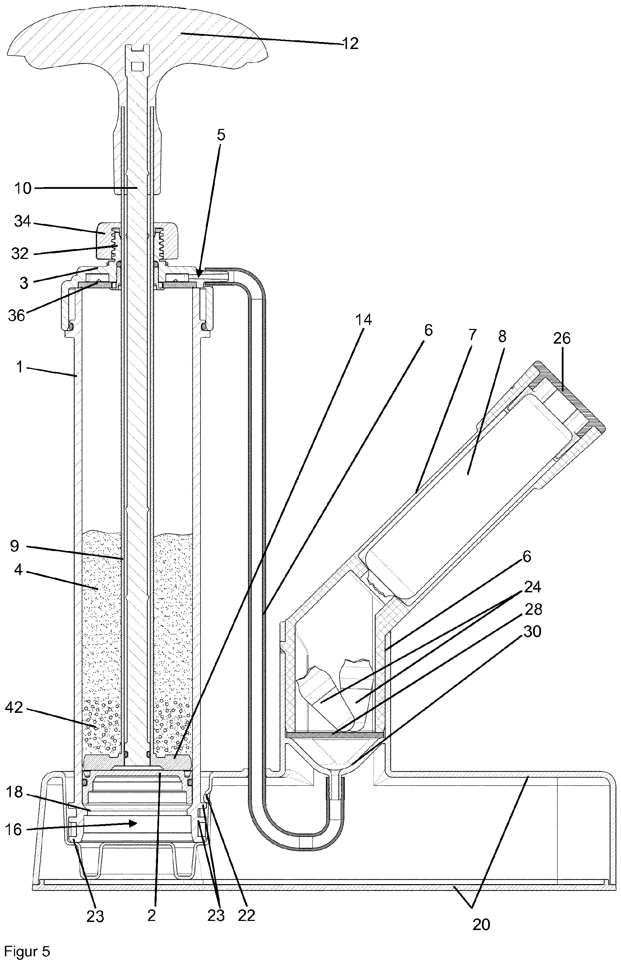

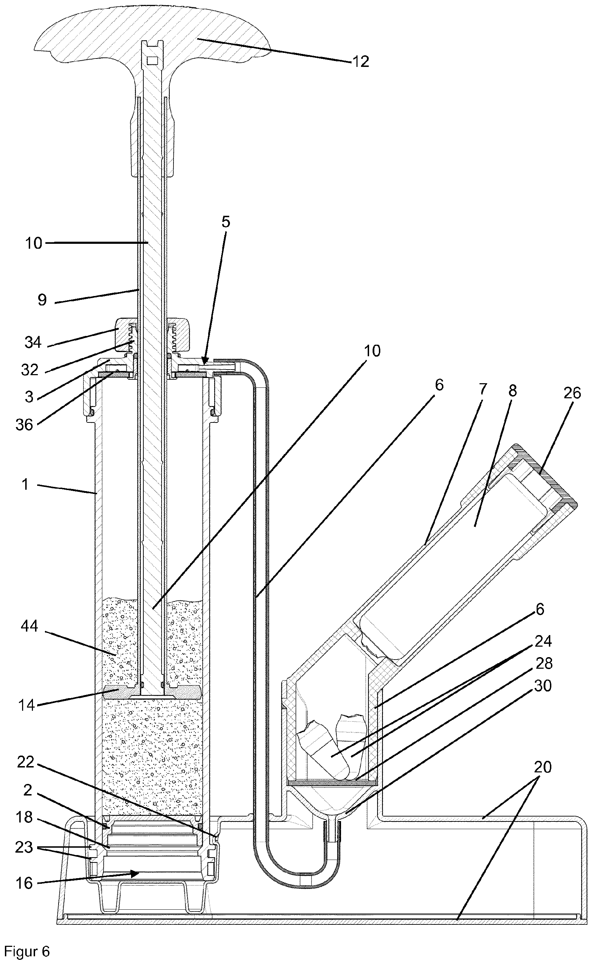

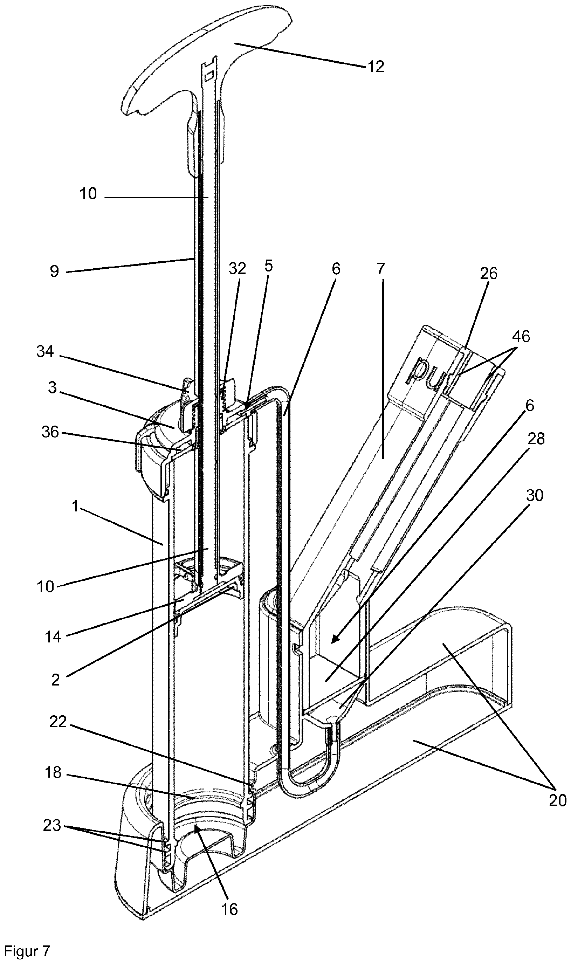

Um im Innenraum der Kartusche 1 einen Unterdruck zu erzeugen, kann der Kolben 2 in Richtung einer Rückseite 16 der Kartusche 1 (bodenseitig, das heißt in den Figuren 1 bis 7 nach unten) beziehungsweise in Richtung der Rückseite 16 des Innenraums der Kartusche 1 bewegt werden, indem der Kolben 2 mit Hilfe des Mischstabs 9 manuell in Richtung der Rückseite 16 gedrückt wird. Damit der Kolben 2 nicht bodenseitig aus dem Innenraum der Kartusche 1 herausgedrückt werden kann, ist an der Innenseite der Kartusche 1 im Bereich der Rückseite 16 ein Anschlag 18 in Form einer umlaufenden Erhebung vorgesehen. Der Kolben 2 kann also nur bis zum Anschlag 18 in Richtung der Rückseite 16 der Kartusche 1 gedrückt werden (siehe Figuren 5 und 6).

Die Kartusche 1 ist lösbar mit einem Fußteil 20 verbunden. Hierzu wird die Kartusche 1 bodenseitig, also im Bereich der Rückseite 16 mit einer Rastnase 22 mit dem Fußteil 20 gerastet. An der Rückseite 16 der Kartusche 1 sind hierzu außen zwei umlaufende Vorsprünge 23 angeordnet. Diese Vorsprünge 23 können zudem zur Befestigung eine Auspressvorrichtung (nicht gezeigt) verwendet werden, mit der der fertig gemischte Knochenzementteig 44 (siehe Figur 6) appliziert werden kann.

Die Glasampulle 8 weist einen abbrechbaren Ampullenkopf 24 auf. Wenn der Ampullenkopf 24 abgebrochen oder aufgebrochen wird, wird damit die Glasampulle 8 geöffnet. Der Behälter 7 ist aus einem elastisch verformbaren Material gefertigt, das eine Verdickung am Hals zwischen dem Ampullenkopf 24 und dem Körper der Ampulle 8 aufweist. Dadurch ist der Ampullenkopf 24 fest gelagert, während der Körper der Ampulle 8 aufgrund der Elastizität des den Behälter 7 bildenden Einsatzes bewegt werden kann. So kann die Glasampulle 8 innerhalb der Lager- und Mischvorrichtung aufgebrochen werden. An der Rückseite des Behälters 7 ist ein Stopfen 26 als Deckel 26 vorgesehen, mit dem die Glasampulle 8 in Position gehalten wird.

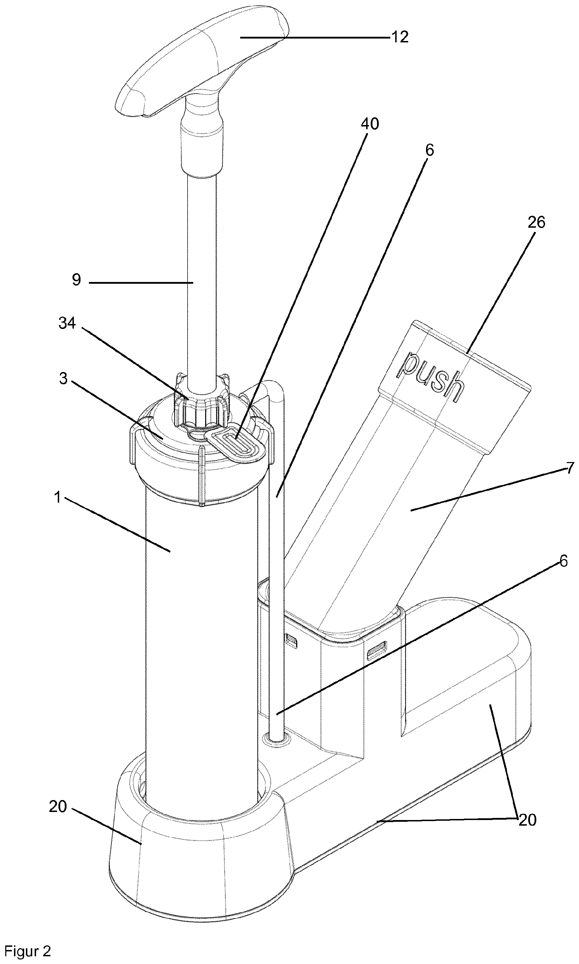

Im verbreiterten Bereich der Fluidleitung 6 unterhalb des Behälters 7 (also in der Monomerflüssigkeitsaufnahme) ist ein Filter 28 und/oder Sieb 28 angeordnet, mit dem Bruchstücke beziehungsweise Glassplitter der Glasampulle 8, die beim Aufbrechen der Glasampulle 8 entstehen können, zurückgehalten werden. Unterhalb der Filters 28 und/oder Siebs 28 verjüngt sich die Fluidleitung 6 über einen Trichter 30, mit dem die Monomerflüssigkeit 42 (siehe Figuren 2 bis 6) in den dünnen Bereich der Fluidleitung 6 geleitet wird.

Im Bereich der Austragsöffnung ist am Kartuschenkopf 3 ein Stutzen 32 mit einem Außengewinde vorgesehen. Auf dieses Außengewinde kann eine konische Überwurfmutter 34 als Befestigungseinrichtung 34 aufgeschraubt werden, um den Mischstab 9 gegen den Kartuschenkopf 3 zu fixieren. Um den Mischstab 9 gegen den Kartuschenkopf 3 fixieren zu können, wird die Überwurfmutter 34 weiter auf das Außengewinde des Stutzens 32 geschraubt. Um dies zunächst zu verhindern, beziehungsweise um eine ungewollte Fixierung zu vermeiden, kann eine herausziehbare Sicherung (nicht gezeigt) in Form eine Spange mit einer Greiflasche vorgesehen, die zwischen der Überwurfmutter 34 und dem Kartuschenkopf 3 angeordnet ist. Wenn die Sicherung abgezogen wird, kann die Überwurfmutter 34 weiter auf das Außengewinde des Stutzens 32 geschraubt werden, so dass der Stutzen 32 zusammengedrückt wird und dadurch den Mischstab 9 gegen den Kartuschenkopf 3 fixiert.

Eine zusätzliche seitliche Öffnung ist im Kartuschenkopf 3 vorgesehen, durch die der Innenraum der Kartusche 1 für sterilisierende Gase, wie Ethylenoxid zugänglich ist. Vorliegend ist die seitliche Öffnung mit einem Verschluss 40 verschlossen.

Zwischen dem Kartuschenkopf 3 und dem Innenraum der Kartusche 1 ist eine Porenscheibe 36 beziehungsweise ein Porenfilter 36 angeordnet, der für Gase und Flüssigkeiten durchlässig ist aber für Pulver und Feststoffe wie das Knochenzementpulver 4 undurchlässig ist, so dass die Durchführung 5 nur über den Porenfilter 36 mit dem restlichen Innenraum der Kartusche 1 verbunden ist. Der Porenfilter 36 verhindert ein Vordringen des Knochenzementpulvers 4 in die Fluidleitung 6 und damit eine ungewollte vorzeitige Reaktion des Knochenzementpulvers 4 mit der Monomerflüssigkeit 42 (siehe Figuren 2 bis 6) und damit einen ungewollten Verschluss des dünnen Teils der Fluidleitung 6.

Anhand der Figuren 1 bis 6 wird im Folgenden ein exemplarisches erfindungsgemäßes Verfahren vorgestellt, das mit der erfindungsgemäßen Lager- und Mischvorrichtung durchgeführt wird. Die fertig aufgebaute Lager- und Mischvorrichtung wird so, wie sie in Figur 1 und 2 gezeigt ist, bereitgestellt. Die Glasampulle 8 wird geöffnet, indem der Ampullenkopf 24 abgebrochen wird, so dass die Monomerflüssigkeit 42 aus der Glasampulle 8 in den verbreiterten Bereich der Fluidleitung 6 hinein ausläuft und über den Trichter 30 auch in den dünnen Bereich der Fluidleitung 6 fließt. Dabei werden Bruchstücke des Glases und der abgebrochene Ampullenkopf 24 durch das Sieb 28 und/oder den Filter 28 zurückgehalten. Die Monomerflüssigkeit 42 aus der Glasampulle 8 ist dann in die Fluidleitung 6 eingefüllt und steht zur Anwendung bereit. Diese Situation ist in Figur 3 gezeigt.

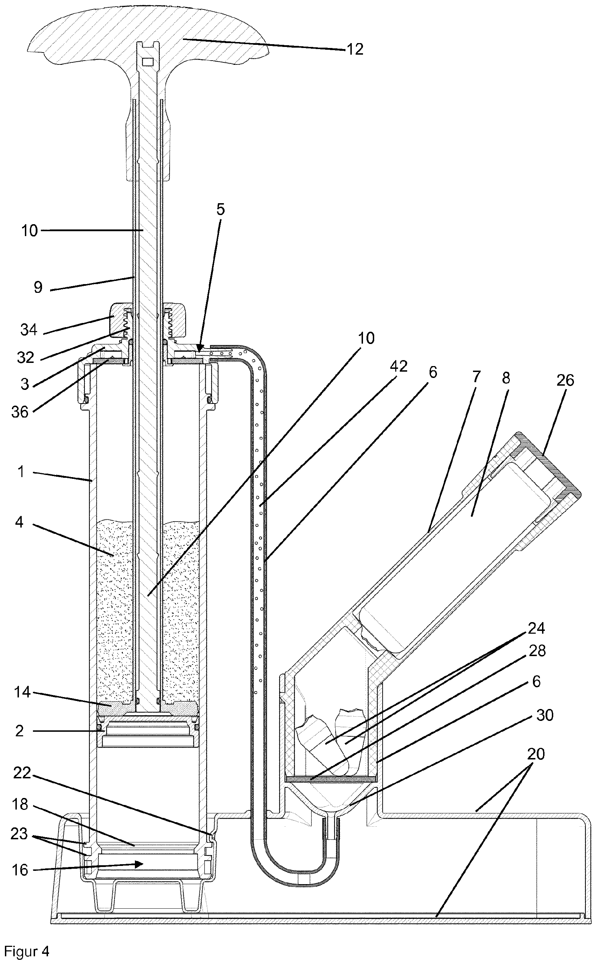

Um die Monomerflüssigkeit 42 in den Innenraum der Kartusche 1 zu transferieren und um im Innenraum der Kartusche 1 einen Unterdruck zur Vermeidung von Gaseinschlüssen im Knochenzementteig 44 (siehe Figur 6) zu erzeugen, wird der Kolben 2 mit Hilfe des Mischstabs 9 in Richtung der Rückseite 16 der Kartusche 1 gedrückt. Dabei vergrößert sich das Volumen zwischen dem Kolben 2 und dem Kartuschenkopf 3 im Innenraum der Kartusche 1, so dass ein Unterdruck entsteht. Der Unterdruck saugt die Monomerflüssigkeit 42 aus der Fluidleitung 6 in Richtung der Kartusche 1 (siehe Figur 4) und schließlich über die Durchführung 5 und den Porenfilter 30 in die Kartusche 1 hinein (siehe Figur 5). Der Kolben 2 ist zu Beginn (siehe Figur 1) derart weit von der Rückseite 16 beziehungsweise dem Anschlag 18 beabstandet, dass der vollständige Hub des Kolbens 2 bis zum Anschlag 18 dazu ausreicht, dass Luft aus der Fluidleitung 6 bis zur Monomerflüssigkeit 42 abgesaugt werden kann und die Monomerflüssigkeit 42 in den Innenraum der Kartusche 1 zu saugen ist.

Der Behälter 7 weist an der Rückseite, zum Beispiel im Deckel 26, Öffnungen 46 auf, so dass Luft von außen in die Fluidleitung 6 nachströmen kann. Die Öffnungen können aber auch sehr klein gestaltet sein, so dass sie in den Figuren nicht erkennbar sind. Dann ist in dem Innenraum der Kartusche 1 jedoch kein Unterdruck mehr vorhanden, wenn die Monomerflüssigkeit 42 vollständig in den Innenraum der Kartusche 1 eingesaugt wurde. Alternativ kann auch eine Restmenge der Monomerflüssigkeit 42 in der Fluidleitung 6 verbleiben. Der Gasdruck im Behälter 7 kann jedoch bei geeignetem Aufbau auch bereits dazu ausreichen, die Monomerflüssigkeit 42 in den Innenraum der Kartusche 1 zu drücken, so dass keine Öffnungen im Behälter 7 benötigt werden.

Wenn die Monomerflüssigkeit 42 in den Innenraum der Kartusche 1 gesaugt ist, kann sie mit Hilfe der Mischeinrichtung 14 mit dem Knochenzementpulver 4 gemischt werden, indem die Mischeinrichtung 14 mit Hilfe des Mischtabs 9 manuell in Längsrichtung axial bewegt und im Innenraum der Kartusche 1 gedreht wird. Dabei entsteht der fertig gemischte Knochenzementteig 44 (siehe Figur 6). Anschließend wird die Mischeinrichtung 14 mit Hilfe des Mischstabs 9 bis zum Anschlag an den Kartuschenkopf 3 gezogen, gegebenenfalls eine Sicherung (nicht gezeigt) entfernt und der Mischstab 9 mit der Überwurfmutter 34 am Kartuschenkopf 3 fixiert. Der Kern 10 wird mit Hilfe des Griffs 12 aus dem Mischstab 9 herausgezogen. Der Mischstab 9 bildet nun ein in der Austragsöffnung angeordnetes beziehungsweise durch die Austragsöffnung geführtes Austragsrohr 9, durch das der fertig gemischte Knochenzementteig 44 appliziert werden kann.

Hierzu wird die Kartusche 1 vom Fußteil 20 getrennt, indem sie herausgezogen wird und die Fluidleitung 6 von der Durchführung 5 abgezogen wird. In der Fluidleitung kann ein Rückschlagventil (nicht gezeigt) vorgesehen sein, um ein Austreten der restlichen Monomerflüssigkeit 42 aus der Fluidleitung 6 zu verhindern. Die Kartusche 1 wird dann in eine Auspressvorrichtung (nicht gezeigt) eingesetzt, mit der der Kolben 2 als Austragskolben 2 mit Hilfe einer vortreibbaren Stange in Richtung des Kartuschenkopfs 3 gepresst werden kann. Dabei wird der Knochenzementteig 44 durch das Austragsrohr 9 und durch die Austragsöffnung ausgepresst. Durch die Durchführung 5 kann kein Knochenzementteig 44 entweichen, da der Porenfilter 36 den Knochenzementteig 44 zurückhält.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Kartusche
- 2: Kolben
- 3: Kartuschenkopf
- 4: Knochenzementpulver / erste Ausgangskomponente
- 5: Durchführung
- 6: Fluidleitung
- 7: Behälter
- 8: Monomerflüssigkeitsbehälter / Glasampulle
- 9: Mischstab / Austragsrohr
- 10: Kern
- 12: Griff
- 14: Mischflügel / Mischeinrichtung
- 16: Rückseite der Kartusche / Bodenseite der Kartusche
- 18: Anschlag
- 20: Fußteil
- 22: Rastnase
- 23: Vorsprung
- 24: Ampullenkopf
- 26: Deckel / Stopfen
- 28: Sieb / Filter
- 30: Trichter
- 32: Stutzen mit Außengewinde
- 34: konische Überwurfmutter / Befestigungseinrichtung
- 36: Porenfilter / Porenscheibe
- 40: Verschluss
- 42: Monomerflüssigkeit
- 44: gemischter Knochenzementteig
- 46: Öffnung

## Patentansprüche

1. Lager- und Mischvorrichtung für Zweikomponenten-Polymethylmethacrylat-Knochenzemente, die Lager- und Mischvorrichtung aufweisend
eine Kartusche (1) mit einem zylindrischen Innenraum, wobei der zylindrische Innenraum an einer Vorderseite durch einen Kartuschenkopf (3) mit einer geschlossenen Austragsöffnung begrenzt ist,
einen Kolben (2), der axial beweglich im zylindrischen Innenraum der Kartusche (1) angeordnet ist und der von dem Kartuschenkopf (3) beabstandet ist, wobei der Kolben (2) umlaufend an der Innenwand des Innenraums anliegt, so dass der Kolben (2) den Innenraum der Kartusche (1) gasdicht in zwei Bereiche trennt,
eine pulverförmige erste Ausgangskomponente (4) des Knochenzements, die in dem Innenraum zwischen dem Kolben (2) und dem Kartuschenkopf (3) enthalten ist,
eine Durchführung (5), die im Kartuschenkopf (3) oder in dem Zylindermantel der Kartusche (1) zwischen dem Kolben (2) und dem Kartuschenkopf (3) angeordnet ist, wobei die Durchführung (5) mit einer Fluidleitung (6) verbunden ist, in der eine Monomerflüssigkeit (42) als zweite Ausgangskomponente (42) des Knochenzements enthalten ist oder in die eine Monomerflüssigkeit (42) einfüllbar oder einleitbar ist, wobei
der Kolben (2) von einer der Vorderseite gegenüberliegenden Rückseite (16) des zylindrischen Innenraums derart weit beabstandet ist, so dass durch eine Bewegung des Kolbens (2) in Richtung der Rückseite (16) ein Unterdruck in dem Innenraum der Kartusche (1) zwischen dem Kolben (2) und dem Kartuschenkopf (3) erzeugbar ist, wobei mit dem Unterdruck die Monomerflüssigkeit (42) aus der Fluidleitung (6) in den Innenraum der Kartusche (1) zu saugen ist,
**dadurch gekennzeichnet, dass** ein Filter (36) im Kartuschenkopf (3), in der Durchführung (5) und/oder in der Fluidleitung (6) angeordnet ist, wobei der Filter (36) für die Monomerflüssigkeit (42) durchlässig und für die erste Ausgangskomponente (4) undurchlässig ist.

2. Lager- und Mischvorrichtung nach Anspruch 1, **dadurch gekennzeichnet, dass**
der Kolben (2) mit einem Stab (9) oder mit einem anderen Kraftübertragungsmittel in dem Innenraum der Kartusche (1) manuell in Richtung der Rückseite (16) des Innenraums zu drücken oder zu ziehen ist, so dass im Innenraum der Kartusche (1) zwischen dem Kolben (2) und dem Kartuschenkopf (3) ein Unterdruck entsteht, wobei vorzugsweise an dem Stab (9) oder an dem Kraftübertragungsmittel ein Betätigungsmittel oder ein Griff (12) zum Bedienen des Stabs (9) oder des Kraftübertragungsmittels befestigt ist.

3. Lager- und Mischvorrichtung nach einem der vorangehenden Ansprüche, **dadurch gekennzeichnet, dass**
der Kolben (2) derart von der Rückseite (16) des zylindrischen Innenraums beabstandet ist, dass der Hubraum des Kolbens (2) bis zu der Rückseite (16) zumindest genauso groß ist, wie das Volumen der einzusaugenden Monomerflüssigkeit (42), vorzugsweise zumindest genauso groß ist, wie das Volumen der Fluidleitung (6) und eines Monomerflüssigkeitsbehälters (8), in dem die Monomerflüssigkeit (42) enthalten ist.

4. Lager- und Mischvorrichtung nach einem der vorangehenden Ansprüche, **dadurch gekennzeichnet, dass**
die Lager- und Mischvorrichtung einen von der Kartusche (1) separaten Behälter (7) aufweist, in dem die Monomerflüssigkeit (42) enthalten ist, wobei der Behälter (7) mit der Fluidleitung (6) verbunden oder verbindbar ist, wobei vorzugsweise in dem Behälter (7) eine geschlossene Glasampulle (8) enthaltend die Monomerflüssigkeit (42) angeordnet oder anzuordnen ist, wobei die Glasampulle (8) innerhalb des Behälters (7) aufbrechbar ist.

5. Lager- und Mischvorrichtung nach Anspruch 4, **dadurch gekennzeichnet, dass**
die Kartusche (1), der separate Behälter (7) und die Fluidleitung (6) mit einem gemeinsamen Standfuß (20) verbunden sind, wobei der Behälter (7) und die Fluidleitung (6) fest mit dem Standfuß (20) verbunden sind und die Kartusche (1) lösbar mit dem Standfuß (20) verbunden ist, bevorzugt die Kartusche (1) über ein Gewinde an den Standfuß (20) geschraubt ist oder über eine Rastung (22) mit dem Standfuß (20) verbunden ist.

6. Lager- und Mischvorrichtung nach Anspruch 4 oder 5, **dadurch gekennzeichnet, dass** im Kartuschenkopf (3), in der Durchführung (5) und/oder in der Fluidleitung (6) ein Filter (36), insbesondere ein Porenfilter (36), angeordnet ist, wobei der Filter (36) für die Monomerflüssigkeit (42) durchlässig und für die erste Ausgangskomponente (4), insbesondere für Zementpulver (4), undurchlässig ist, und/oder
in dem separaten Behälter (7) ein Monomerflüssigkeitsbehälter (8) enthalten ist, insbesondere ein Folienbeutel oder eine Glasampulle (8) enthalten ist, der innerhalb des separaten Behälters (7) zu öffnen ist, so dass die Monomerflüssigkeit (42) aus dem geöffneten Monomerflüssigkeitsbehälter (8) in die Fluidleitung (6) fließt, wobei vorzugsweise am Behälter (7) eine von außen bedienbare Öffnungsvorrichtung zum Öffnen des Monomerflüssigkeitsbehälters (8) angeordnet ist.

7. Lager- und Mischvorrichtung nach einem der vorangehenden Ansprüche, **dadurch gekennzeichnet, dass**
die Lager- und Mischvorrichtung Öffnungen (46) aufweist, durch die Luft von außen in die Fluidleitung (6) nachströmt, wobei die Öffnungen (46) vorzugsweise in einem Behälter (7) für die Monomerflüssigkeit (42) angeordnet sind, besonders bevorzugt in einem Deckel (26) an der Rückseite des Behälters (7) angeordnet sind.

8. Lager- und Mischvorrichtung nach einem der vorangehenden Ansprüche, **dadurch gekennzeichnet, dass**
in dem Innenraum der Kartusche (1) zwischen dem Kolben (2) und dem Kartuschenkopf (3) eine Mischeinrichtung (14) angeordnet ist, mit der die erste Ausgangskomponente (4) mit der Monomerflüssigkeit (42) im Innenraum der Kartusche (1) zu mischen ist.

9. Lager- und Mischvorrichtung nach Anspruch 8, **dadurch gekennzeichnet, dass**
die Mischeinrichtung (14) über einen Mischstab (9) von außerhalb der Kartusche (1) bedienbar ist, wobei der Mischstab (9) gasdicht durch den Kartuschenkopf (3) geführt ist sowie drehbar und in axialer Richtung beweglich ist, vorzugsweise der Mischstab (9) durch die Austragsöffnung geführt ist.

10. Lager- und Mischvorrichtung nach Anspruch 9, **dadurch gekennzeichnet, dass**
der Kolben (2) mit dem Mischstab (9) in Richtung der Rückseite (16) des Innenraums der Kartusche (1) zu drücken ist.

11. Lager- und Mischvorrichtung nach einem der Ansprüche 9 oder 10, **dadurch gekennzeichnet, dass**
der Mischstab (9) ein Austragsrohr (9) ist, in dem ein manuell entfernbarer Kern (10) angeordnet ist, so dass der gemischte Knochenzementteig (44) aus dem Innenraum der Kartusche (1) durch das Austragsrohr (9) ohne den Kern (10) auszutreiben ist.

12. Lager- und Mischvorrichtung nach einem der vorangehenden Ansprüche, **dadurch gekennzeichnet, dass**
der Kolben (2) mindestens ein Rastelement besitzt, das mit einer Gegenrastung an der Innenseite der Kartusche (1) im Bereich der Rückseite (16) des Innenraums lösbar zu rasten ist und/oder im Innenraum der Kartusche (1) an der Rückseite (16) ein Anschlag (18) vorgesehen ist, der die Bewegung des Kolbens (2) in Richtung Rückseite (16) begrenzt.

13. Lager- und Mischvorrichtung nach einem der vorangehenden Ansprüche, **dadurch gekennzeichnet, dass**
im Kartuschenkopf (3) eine gasdurchlässige Öffnung, insbesondere eine verschließbare gasdurchlässige Öffnung, angeordnet ist, wobei eine für Gase durchlässige und für Feststoffpartikel undurchlässige poröse Scheibe (36) zwischen der ersten Ausgangskomponente (4) und der Öffnung angeordnet ist, wobei bevorzugt die poröse Scheibe (36) im Kartuschenkopf (3) angeordnet ist.

14. Verfahren zur Vermischung der Ausgangskomponenten (4, 42) eines Knochenzements, insbesondere eines Mehrkomponenten-Polymethylmethacrylat-Knochenzements, mit einer Lager- und Mischvorrichtung nach mindestens einem der vorangehenden Ansprüche, **gekennzeichnet durch** die folgenden Schritte,
a) mit dem Filter (36) wird ein Vordringen der ersten Ausgangskomponente (4) in die Fluidleitung (6) verhindert,
b) der Kolben (2) wird in Richtung der Rückseite (16) des zylindrischen Innenraums der Kartusche (1) bewegt, wobei durch die Bewegung des Kolbens (2) im Innenraum der Kartusche (1) zwischen dem Kolben (2) und dem Kartuschenkopf (3) ein Unterdruck entsteht,
c) die Monomerflüssigkeit (42) wird mit dem Unterdruck im Innenraum aus der Fluidleitung (6) und durch den Filter (36) in den Innenraum der Kartusche (1) gesaugt,
d) die Monomerflüssigkeit (42) und die erste Ausgangskomponente (4) werden im Innenraum der Kartusche (1) zu dem Knochenzementteig (44) gemischt, und
e) der Knochenzementteig (44) wird durch Vortreiben des Kolbens (2) in Richtung des Kartuschenkopfs (3) aus dem Innenraum der Kartusche (1) durch die geöffnete Austragsöffnung ausgetrieben.

15. Verfahren nach Anspruch 14, **dadurch gekennzeichnet, dass**
vor Schritt b) die Monomerflüssigkeit (42) in die Fluidleitung (6) eingeleitet wird, insbesondere nach Öffnen eines Monomerflüssigkeitsbehälters (8) in der Lager- und Mischvorrichtung.

16. Verfahren nach einem der Ansprüche 14 oder 15, **dadurch gekennzeichnet, dass** in Schritt b) der Kolben (2) mit einem Mischstab (9) in Richtung der Rückseite (16) der Kartusche (1) gedrückt wird, wobei der Mischstab (9) beweglich in einer gasdichten Durchführung, insbesondere in der Austragsöffnung, im Kartuschenkopf (3) gelagert ist, und in Schritt d) die Mischung der Monomerflüssigkeit (42) mit der ersten Ausgangskomponente (4) durch Bewegen einer mit dem Mischstab (9) verbundenen Mischeinrichtung (14) im Innenraum der Kartusche (1) zwischen dem Kolben (2) und dem Kartuschenkopf (3) erfolgt, indem der Mischstab (9) und damit die Mischeinrichtung (14) bewegt wird.

17. Verfahren nach Anspruch 16, **dadurch gekennzeichnet, dass**
zwischen Schritt d) und e) die Mischeinrichtung (14) mit dem Mischstab (9) zum Kartuschenkopf (3) gezogen wird und ein Kern (10) aus dem Mischstab (9) entfernt wird, so dass der Mischstab (9) ohne den Kern (10) ein Austragsrohr (9) bildet, durch das der gemischte Knochenzementteig (44) in Schritt e) aus dem Innenraum der Kartusche (1) ausgepresst wird und /oder vor Schritt e) die Kartusche (1) von der Lager- und Mischvorrichtung getrennt wird und in eine Auspressvorrichtung eingesetzt wird, mit der in Schritt e) der Kolben (2) mittels eines Stößels oder einer vortreibbaren Stange in Richtung des Kartuschenkopfs (3) gedrückt wird, um den Knochenzementteig (44) aus dem Innenraum der Kartusche (1) auszutreiben, wobei vorzugsweise vor Schritt b) der Kartuschenkopf (3) gasdicht verschlossen wird.

18. Verfahren nach einem der Ansprüche 14 bis 17, **dadurch gekennzeichnet, dass**
in Schritt c) Luft von außerhalb der Lager- und Mischvorrichtung durch Öffnungen (46) in einen Behälter (7) für die Monomerflüssigkeit (42) nachströmt.

## Claims

1. A storage and mixing device for two-component polymethyl methacrylate bone cements, said storage and mixing device having
a cartridge (1) with a cylindrical interior, wherein the cylindrical interior is delimited on a front side by a cartridge head (3) with a closed delivery opening,
a plunger (2) which is arranged to be axially movable in the cylindrical interior of the cartridge (1) and which is spaced from the cartridge head (3), wherein the plunger (2) circumferentially rests against the inner wall of the interior, so that the plunger (2) divides the interior of the cartridge (1) into two sections in a gas-tight manner,
a powdery first parent component (4) of the bone cement, which is contained in the interior between the plunger (2) and the cartridge head (3),
a feed-through (5) which is arranged in the cartridge head (3) or in the cylinder barrel of the cartridge (1) between the plunger (2) and the cartridge head (3), wherein the feed-through (5) is connected to a fluid line (6) in which a monomer liquid (42) as a second parent component (42) of the bone cement is contained or into which a monomer liquid (42) is fillable or introducible, wherein
the plunger (2) is spaced from a back side (16) of the cylindrical interior, located opposite the front side, to an extent that, by moving the plunger (2) in the direction of the back side (16), a reduced pressure is producible in the interior of the cartridge (1) between the plunger (2) and the cartridge head (3), wherein the reduced pressure is able to suck the monomer liquid (42) out of the fluid line (6) into the interior of the cartridge (1),
**characterized in that** a filter (36) which is arranged in the cartridge head (3), in the feed-through (5) and/or in the fluid line (6), wherein the filter (36) is permeable to the monomer liquid (42) and impermeable to the first parent component (4).

2. The storage and mixing device according to Claim 1, **characterized in that**
the plunger (2) in the interior of the cartridge is pushable or pullable manually in the direction of the back side (16) of the interior by means of a rod (9) or another force transmission means, so that a reduced pressure is produced in the interior of the cartridge (1) between the plunger (2) and the cartridge head (3), wherein preferably an actuating means or a handle (12) for operating the rod (9) or the force transmission means is affixed to the rod (9) or the force transmission means.

3. The storage and mixing device according to any one of the preceding claims, **characterized in that**
the plunger (2) is spaced from the back side (16) of the cylindrical interior such that the space for displacement of the plunger (2) to reach the back side (16) is at least equal to the volume of the monomer liquid (42) to be sucked in, preferably at least equal to the volume of the fluid line (6) and a monomer liquid container (8) in which the monomer liquid (42) is contained.

4. The storage and mixing device according to any one of the preceding claims, **characterized in that**
the storage and mixing device has a container (7) which is separate from the cartridge (1) and in which the monomer liquid (42) is contained, wherein the container (7) is connected or connectable to the fluid line (6), wherein preferably a closed glass ampoule (8) containing the monomer liquid (42) is arranged or is arrangeable in the container (7), wherein the glass ampoule (8) is breakable open within the container (7).

5. The storage and mixing device according to Claim 4, **characterized in that**
the cartridge (1), the separate container (7) and the fluid line (6) are connected to a common stand (20), wherein the container (7) and the fluid line (6) are permanently connected to the stand (20) and the cartridge (1) is releasably connected to the stand (20), the cartridge (1) preferably being screwed to the stand (20) by means of a thread or connected to the stand (20) by means of a latching mechanism (22).

6. The storage and mixing device according to Claim 4 or 5 **characterized in that**
a filter (36) is arranged in the cartridge head (3), in the feed-through (5) and/or in the fluid line (6), wherein the filter (36) is permeable to the monomer liquid (42) and impermeable to the first parent component (4), particularly to the cement powder (4), and/or
a monomer liquid container (8), in particular a foil bag or a glass ampoule (8), is contained in the separate container (7), which is openable within the separate container (7), so that the monomer liquid (42) flows out of the opened monomer liquid container (8) into the fluid line (6), wherein preferably an opening device for opening the monomer liquid container (8), which is operable from outside, is arranged on the container (7).

7. The storage and mixing device according to any one of the preceding claims, **characterized in that**
the storage and mixing device has openings (46) through which air flows into the fluid line (6) from outside, wherein the openings are preferably arranged in a container (7) for the monomer liquid (42), particularly preferably in a lid (26) on the back side of the container (7).

8. The storage and mixing device according to any one of the preceding claims, **characterized in that**
a mixing device (14) is arranged in the interior of the cartridge (1) between the plunger (2) and the cartridge head (3), by means of which the first parent component (4) is mixable with the monomer liquid (42) in the interior of the cartridge (1).

9. The storage and mixing device according to Claim 8, **characterized in that** the mixing device (14) is operable from outside the cartridge (1) by means of a mixing rod (9), wherein the mixing rod (9) passes through the cartridge head (3) in a gas-tight manner and is rotatable and movable in the axial direction, the mixing rod (9) preferably passing through the delivery opening.

10. The storage and mixing device according to Claim 9, **characterized in that** the plunger (2) is pushable in the direction of the back side (16) of the interior of the cartridge (1) by means of the mixing rod (9).

11. The storage and mixing device according to any one of Claims 9 or 10, **characterized in that**
the mixing rod (9) is a delivery pipe (9) in which a manually removable core (10) is arranged, so that the mixed bone cement paste (44) is dischargeable from the interior of the cartridge (1) through the delivery pipe (9) without the core (10).

12. The storage and mixing device according to any one of the preceding claims, **characterized in that**
the plunger (2) has at least one latching element which is releasably engageable with a complementary latching element on the inner side of the cartridge (1) in the region of the back side (16) of the interior and/or in the interior of the cartridge (1), on the back side (16), a stop (18) is provided which delimits movement of the plunger (2) in the direction of the back side (16).

13. The storage and mixing device according to any one of the preceding claims, **characterized in that**
a gas-permeable opening, in particular a closable gas-permeable opening, is arranged in the cartridge head (3), wherein a porous disc (36) which is permeable to gases and
impermeable to solid particles is arranged between the first parent component (4) and the opening, wherein the porous disc (36) is preferably arranged in the cartridge head (3).

14. A method for mixing the parent components (4, 42) of a bone cement, in particular a multicomponent polymethyl methacrylate bone cement, using a storage and mixing device according to at least one of the preceding claims, **characterized by** the following steps:
a) the filter (36) prevents the first parent component (4) from making its way into the fluid line (6),
b) the plunger (2) is moved in the direction of the back side (16) of the cylindrical interior of the cartridge (1), wherein a reduced pressure is produced in the interior of the cartridge (1) between the plunger (2) and the cartridge head (3) due to said movement of the plunger (2),
c) the monomer liquid (42) is sucked out of the fluid line (6) and through the filter (36) into the interior of the cartridge (1) by the reduced pressure in the interior,
d) the monomer liquid (42) and the first parent component (4) are mixed to form the bone cement paste (44) in the interior of the cartridge (1), and
e) the bone cement paste (44) is discharged from the interior of the cartridge (1) through the opened delivery opening by advancing the plunger (2) in the direction of the cartridge head (3).

15. The method according to Claim 14, **characterized in that**
the monomer liquid (42) is introduced into the fluid line (6) before step b), in particular after a monomer liquid container (8) has been opened in the storage and mixing device.

16. The method according to any one of Claims 14 or 15, **characterized in that**
in step b), the plunger (2) is pressed in the direction of the back side (16) of the cartridge (1) by means of a mixing rod (9), wherein the mixing rod (9) is movably supported in a gas-tight feed-through, in particular in the delivery opening, in the cartridge head (3), and, in step d), the monomer liquid (42) is mixed with the first parent component (4) by moving a mixing device (14), which is connected to the mixing rod (9), in the interior of the cartridge (1) between the plunger (2) and the cartridge head (3), by moving the mixing rod (9) and thereby the mixing device (14).

17. The method according to Claim 16, **characterized in that**
the mixing device (14) is pulled towards the cartridge head (3) by means of the mixing rod (9) and a core (10) is removed from the mixing rod (9) between steps d) and e), so that the mixing rod (9) without the core (10) forms a delivery pipe (9) through which the mixed bone cement paste (44) is pressed out of the interior of the cartridge (1) in step e) and/or
before step e), the cartridge (1) is separated from the storage and mixing device and inserted into a press-out device by means of which the plunger (2) is pressed in the direction of the cartridge head (3) in step e), using a tappet or an advanceable rod, in order to discharge the bone cement paste (44) from the interior of the cartridge (1), wherein preferably the cartridge head (3) is closed to be gas-tight before step b).

18. The method according to any one of Claims 14 to 17, **characterized in that**
air flows in from outside the storage and mixing device through openings (46) in a container (7) for the monomer liquid (42) in step c).

## Revendications

1. Dispositif de stockage et de mélange pour un ciment osseux en polyméthylméthacrylate bi composant, le dispositif de stockage et de mélange présentant
une cartouche (1) avec un espace intérieur cylindrique, l'espace intérieur cylindrique étant délimité sur le côté frontal par une tête de cartouche (3) avec un orifice de décharge fermé,
un piston (2) qui est disposé mobile axialement dans l'espace intérieur cylindrique de la cartouche (1) et qui espacé par rapport à la tête de cartouche (3), où le piston (2) est plaqué sur la circonférence de la paroi intérieure de l'espace intérieur de sorte que le piston (2) sépare de manière étanche aux gaz l'espace intérieur de la cartouche (1) en deux zones,
une première composante de départ (4) sous forme de poudre du ciment osseux qui est contenue dans l'espace intérieur entre le piston (2) et la tête de cartouche (3),
un passage (5) qui est placé dans la tête de cartouche (3) ou dans l'enveloppe de cylindre de la cartouche (1) entre le piston (2) et la tête de cartouche (3),
où le passage (5) est relié avec une conduite de fluide (6) dans laquelle est contenu un liquide de monomère (42) servant de deuxième composante de départ (42) du ciment osseux ou dans laquelle un liquide de monomère (42) peut être rempli ou peut être alimenté,
où le piston (2) est séparé d'un côté arrière (16) de l'espace intérieur cylindrique situé en face du côté frontal d'une distance telle qu'une dépression peut être générée dans l'espace intérieur de la cartouche (1) entre le piston (2) et la tête de cartouche (3) par un déplacement du piston (2) en direction du côté arrière (16),
où, avec la dépression, le liquide de monomère (42) peut être aspiré hors de la conduite de fluide (6) vers l'espace intérieur de la cartouche (1),
**caractérisé en ce qu'**un filtre (36) est disposé dans la tête de cartouche (3), dans le passage (5) et/ou dans la conduite de fluide (6), où le filtre (36) laisse passer le liquide de monomère (42) et ne laisse pas passer la première composante de départ (4).

2. Dispositif de stockage et de mélange selon la revendication 1, **caractérisé en ce que** le piston (2) peut être pressé ou tiré manuellement avec une barre (9) ou avec un autre moyen de transmission de force dans l'espace intérieur de la cartouche (1) en direction du côté arrière (16) de l'espace intérieur de sorte qu'il se crée une dépression dans l'espace intérieur de la cartouche (1) entre le piston (2) et la tête de cartouche (3), où, de préférence, un moyen d'actionnement ou une poignée (12), pour se servir de la barre (6) ou du moyen de transmission de force, est fixé sur la barre (9) ou sur le moyen de transmission de force.

3. Dispositif de stockage et de mélange selon l'une des revendications précédentes, **caractérisé en ce que**
le piston (2) est espacé par rapport au côté arrière (16) de l'espace intérieur cylindrique de telle manière que la course du piston (2) jusqu'au côté arrière (16) est au moins aussi grande que le volume du liquide de monomère (42) à aspirer, de préférence, au moins aussi grande que le volume de la conduite de fluide (6) et d'un récipient de liquide de monomère (8) dans lequel est contenu le liquide de monomère (42).

4. Dispositif de stockage et de mélange selon l'une des revendications précédentes, **caractérisé en ce que**
le dispositif de stockage et de mélange présente un récipient (7) séparé de la cartouche (1) dans lequel est contenu le liquide de monomère (42), où le récipient (7) est relié ou peut être relié avec la conduite de fluide (6), où, de préférence, une ampoule en verre (8) fermée contenant le liquide de monomère (42) est disposée ou peut être disposée dans le récipient (7), où l'ampoule en verre (8) peut être cassée à l'intérieur du récipient (7).

5. Dispositif de stockage et de mélange selon la revendication 4, **caractérisé en ce que** la cartouche (1), le récipient (7) séparé et la conduite de fluide (6) sont reliés avec un partie de base (20) commune, où le récipient (7) et la conduite de fluide (6) sont reliés de manière fixe avec la partie de base (20) et la cartouche (1) est reliée de manière amovible avec la partie de base (20), de préférence, la cartouche (1) est vissée sur la partie de base (20) par un filetage ou est reliée avec la partie de base (20) par un encliquetage (22).

6. Dispositif de stockage et de mélange selon la revendication 4 ou 5, **caractérisé en ce que**
dans la tête de cartouche (3), dans le passage (5) et/ou dans la conduite de fluide (6) est disposé un filtre (36), notamment un filtre poreux (36), où le filtre (36) laisse passer le liquide de monomère (42) et ne laisse pas passer la première composante de départ (4), notamment pour la poudre de ciment (4), et/ou
dans le récipient (7) séparé est contenu un récipient de liquide de monomère (8), notamment un sachet en film ou une ampoule en verre (8) qui est à ouvrir à l'intérieur du récipient (7) séparé de sorte que le liquide de monomère (42) coule du récipient de liquide de monomère (8) dans la conduite de fluide (6), où, de préférence, un dispositif d'ouverture pouvant être actionné de l'extérieur est disposé sur le récipient (7) pour l'ouverture du récipient de liquide de monomère (8).

7. Dispositif de stockage et de mélange selon l'une des revendications précédentes, **caractérisé en ce que**
le dispositif de stockage et de mélange présente des orifices (46) par lesquels de l'air passe l'air de l'extérieur dans la conduite de fluide (6), où les orifices (46) sont disposés de préférence dans un récipient (7) pour le liquide de monomère (42), de manière particulièrement préférée, sont disposés dans un couvercle (26) sur le côté arrière du récipient (7).

8. Dispositif de stockage et de mélange selon l'une des revendications précédentes, **caractérisé en ce**
**qu'**un système de mélange (14) est disposé dans l'espace intérieur de la cartouche (1) entre le piston (2) et la tête de cartouche (3) avec lequel la première composante de départ (4) est à mélanger avec le liquide de monomère (42) dans l'espace intérieur de la cartouche (1).

9. Dispositif de stockage et de mélange selon la revendication 8, **caractérisé en ce que** le système de mélange (14) peut être actionné de l'extérieur de la cartouche (1) par le biais d'une tige de mélange (9), où la tige de mélange (9) est menée de manière étanche aux gaz à travers la tête de cartouche (3) et est également mobile en pouvant être mise en rotation et en direction axiale, de préférence la tige de mélange (9) est menée à travers l'orifice de décharge.

10. Dispositif de stockage et de mélange selon la revendication 9, **caractérisé en ce que** le piston (2) avec la tige de mélange (9) est à presser en direction du côté arrière (16) de l'espace intérieur de la cartouche (1).

11. Dispositif de stockage et de mélange selon l'une des revendications 9 ou 10, **caractérisé en ce que**
la tige de mélange (9) est un tube de décharge (9) dans lequel est disposé une partie centrale (10) pouvant être enlevée manuellement de sorte que la pâte de ciment osseux (44) mélangée est à expulser de l'espace intérieur de la cartouche (1) par le tube de décharge (9) dépourvu de la partie centrale (10).

12. Dispositif de stockage et de mélange selon l'une des revendications précédentes, **caractérisé en ce que**
le piston (12) possède au moins un élément d'encliquetage qui est à verrouiller de manière amovible avec un élément d'encliquetage complémentaire sur le côté intérieur de la cartouche (1) dans la région du côté arrière (16) de l'espace intérieur, et/ou une butée (18) est prévue dans l'espace intérieur de la cartouche (1) sur le côté arrière (16) qui limite le déplacement du piston (2) en direction du côté arrière (16).

13. Dispositif de stockage et de mélange selon l'une des revendications précédentes, **caractérisé en ce que**
dans la tête de cartouche (3) est disposée un orifice laissant passer les gaz, notamment un orifice laissant passer les gaz pouvant être fermé, où un disque (36) poreux laissant passer les gaz et ne laissant pas passer les particules solides est disposé entre la première composante de départ (4) et l'orifice, où, de préférence, le disque (36) poreux est disposé dans la tête de cartouche (3).

14. Procédé de mélange des composantes de départ (4, 42) d'un ciment osseux, notamment d'un ciment osseux de polyméthylméthacrylate multi composant, avec un dispositif de stockage et de mélange selon au moins l'une des revendications précédentes, **caractérisé par** les étapes suivantes,
a) à l'aide du filtre (36), on empêche une avancée de la première composante de départ (4) dans la conduite de fluide (6),
b) le piston (2) est déplacé en direction du côté arrière (16) de l'espace intérieur cylindrique de la cartouche (1), ce par quoi une dépression est générée par le déplacement du piston (2) dans l'espace intérieur de la cartouche (1) entre le piston (2) et la tête de cartouche (3),
c) le liquide de monomère (42) est aspiré à partir de la conduite de fluide (6) et à travers le filtre (36) dans l'espace intérieur de la cartouche (1) avec la dépression,
d) le liquide de monomère (42) et la première composante de départ (4) sont mélangés dans l'espace intérieur de la cartouche (1) pour avoir une pâte de ciment osseux (44), et
e) la pâte de ciment osseux (44) est poussée hors de l'espace intérieur de la cartouche (1) par l'avancée du piston (2) en direction de la tête de cartouche (3) par l'orifice de décharge ouvert.

15. Procédé selon la revendication 14, **caractérisé en ce**
**qu'**avant l'étape b), le liquide de monomère (42) est alimenté dans la conduite de fluide (6), notamment après l'ouverture d'un récipient de liquide de monomère (6) dans le dispositif de stockage et de mélange.

16. Procédé selon l'une des revendications 14 ou 15, **caractérisé en ce que**
dans l'étape b), le piston (2) est pressé avec une tige de mélange (9) en direction du côté arrière (16) de la cartouche (1), où la tige de mélange (9) est logée dans la tête de cartouche (3), mobile dans un passage étanche aux gaz, notamment dans l'orifice de décharge, et dans l'étape d) le mélange du liquide de monomère (4) avec la première composante de départ (4) a lieu par le déplacement d'un système de mélange (14) relié avec la tige de mélange (9) dans l'espace intérieur de la cartouche (1) entre le piston (2) et la tête de cartouche (3) **en ce que** la tige de mélange (9) et par conséquent le système de mélange (14) sont agités.

17. Procédé selon la revendication 16, **caractérisé en ce**
**qu'**entre l'étape d) et l'étape e), le système de mélange (14) est tiré vers la tête de cartouche (3) avec la tige de mélange (9) et une partie centrale (10) est enlevée de la tige de mélange (9) de sorte que la tige de mélange (9) dépourvue de la partie centrale (10) constitue le tube de décharge (9), par lequel la pâte de ciment osseux (44) mélangée dans l'étape e) est pressée hors de l'espace intérieur de la cartouche (1) et/ou avant l'étape e), la cartouche (1) est séparée du dispositif de stockage et de mélange et est mise en service dans un dispositif de pressage avec lequel, dans l'étape e), le piston (2) est pressé au moyen d'un poussoir ou d'une tige pouvant avancer en direction de la tète de cartouche (3) afin d'expulser la pâte de ciment osseux (44) hors de l'espace intérieur de la cartouche (1), où la tête de cartouche (3) est fermée de manière étanche aux gaz de préférence avant l'étape b).

18. Procédé selon l'une des revendications 14 à 17, **caractérisé en ce que**
dans l'étape c) de l'air provenant de l'extérieur du dispositif de stockage et de mélange s'écoule après pour le liquide de monomère (42) par des orifices (46) dans un récipient (7).
